# EUROPEAN PATENT APPLICATION

(11) **EP 3 888 628 A1**
(43) Date of publication of application: **06.10.2021**
(21) Application number: 21170549.6
(22) Date of filing: 15.03.2013
(51) Int. Cl.: A61K 8/99, A61P 31/04, A61P 17/00, A01N 63/20, A61Q 19/08, A61P 31/00, A61K 9/00, A61K 35/745, A61K 35/747

(54) **COSMETIC USE OF A PROBIOTIC BACTERIA LYSATE**

(30) Priority: 13.04.2012 GB 201206599
(62) Divisional of application: 13711111.8
(71) Applicant: Skinbiotherapeutics PLC, Park Green, Macclesfield SK11 7QJ (GB)
(72) Inventor: O'NEILL, Catherine, Manchester, M13 9PL (GB); McBAIN, Andrew, Manchester, M13 9PL (GB)
(74) Representative: Appleyard Lees IP LLP

(57) **Abstract**

The invention provides probiotic bacteria lysates for cosmetic treatment. Methods involving regeneration or repair of the skin barrier are contemplated.

## Description

### Field of the Invention

The present invention relates to probiotic bacteria and particularly, although not exclusively, to medical and cosmetic applications for probiotic bacteria and lysates thereof.

### Background to the Invention

Humans live in constant contact with a multitude of micro-organisms. The gut is by far the most heavily colonised and the gut microbiota has been shown to play multiple roles in normal physiology including nutrient sequestration (3), and development of normal immune responses (22).

Amongst the normal gut microbiota are the so-called probiotic bacteria. Ingestion of these has been shown to prevent or treat gastrointestinal disorders such as antibiotic-associated diarrhoea (47) and inflammatory bowel disease (42). The mechanisms underlying these effects are largely unknown. However, studies have suggested that probiotics can inhibit colonisation of the gut by pathogens. Work *in vitro* has suggested that probiotics use various mechanisms to inhibit pathogens including direct competition for binding sites on epithelial cells (12) and competition with pathogenic bacteria for nutrients. Probiotic organisms are also able to produce inhibitory substances such as bacteriocins (13) and organic acids (25) that can kill or limit the growth of pathogens. Selected probiotics such as *L. plantarum* 299v have been shown to upregulate mucin production by epithelial cells thereby preventing pathogen attachment (33). Probiotics may also produce biosurfactants that allow attachment of the probiotic while inhibiting attachment of pathogenic bacteria to cells (43).

In contrast to the gut, very little is known about the normal interactions between the skin microflora and the epidermis. Recent work suggests that skin commensals may also be able to limit the colonisation of the skin by pathogenic bacteria (48). Studies also suggest that certain skin diseases (such as acne vulgaris and atopic dermatitis) may be associated with disruptions to the normal microflora (4, 6, 8). Therefore, the idea that the skin microflora may be modulated using specific skin commensals to promote health or inhibit disease has received some attention (30, 31). However, the skin commensal bacteria can also be pathogenic under certain circumstances (16). By contrast probiotics are generally regarded as safe (GRAS) and therefore, these bacteria could potentially be used topically if they have therapeutic value (17). So far the limited amount of research in this area suggests that conventional probiotic bacteria may be of significant value when used on the skin. For example, topical application of a B. *longum reuter* lysate has been shown to induce clinical improvement of "reactive skin". This is skin that is more sensitive to physical changes such as atmospheric temperature, and to chemical changes such as seen with topically applied products (20). Application of the B. *longum* lysate to the skin of volunteers was shown to decrease sensitivity and decrease transepidermal water loss (TEWL) after tape stripping. Additionally, application of the lysate to *ex-vivo* skin was shown to decrease signs of inflammation such as vasodilation, oedema and TNF-α release (20). Topical application of *L. plantarum* has also been demonstrated to improve tissue repair in a burned mouse model and prevent infection in chronic leg ulcers and burns in humans (40, 41, 46). However, in general, the mechanisms underlying these effects are unknown.

*Staphylococcus aureus* is a transient coloniser of skin predominantly in the moist, warm regions of the body such as the groin, axilla and the anterior nares (28). Up to 60% of people are intermittent carriers while 20% of people may be stably colonised (28). While normal carriage is asymptomatic, S. *aureus* may invade tissues (e.g. through broken skin) where it causes diseases ranging from the relatively minor impetigo and scalded skin syndrome, to life threatening conditions such as septicaemia (29). Furthermore, S. *aureus* infection is often a secondary phenomenon in skin with underlying conditions such as atopic dermatitis (27).

Tight junctions (TJs) are multi protein complexes sealing the paracellular space between adjacent epithelial cells and limiting transport through this pathway to small, hydrophilic molecules and ions (55). Transepithelial electrical resistance (TEER) is a measure of TJ function (56, 57). TJ function is often reflected in the expression levels of particular proteins involved in the complexes. The main TJ proteins expressed by keratinocytes include claudin 1, claudin 4, ZO-1 and occludin.

Changes in the expression levels of claudins in particular have been shown many times previously to be linked to changes in barrier function. To date 24 mammalian claudins have been identified and these generally fall into two classes - those which strengthen the barrier and those which form selective pores (58). Several lines of evidence point to a role for claudins 1 and 4 as barrier strengthening claudins. In cell lines, overexpression of claudin 1 increased the TEER and decreased the permeability of cells to paracellular markers. Claudin 4 seals the paracellular space against the passage of ions and in doing so increases the TEER of monolayers [59, 60]. Increases in ZO-1 expression enhance the TEER of A431 cells (61) and the hormone GLP1 also enhances TJ function in caco-2 cells by increasing ZO-1 and occludin expression (62). The existence of TJs in skin has been discovered only relatively recently. Therefore, at present the contribution of particular TJ protein species to skin barrier function is largely unknown. However, genetic loss of claudin 1 is known to be lethal in mice (63).

Keratinocytes sense the presence of bacteria via pattern recognition receptors such as toll like receptors (TLRs). Several lines of evidence in the gut have pointed to a relationship between TLR activation and changes in TJ barrier function. Recent work by Yuki et al. (53) demonstrated TLR-mediated augmentation of TJ function in keratinocytes in response to bacterial ligands such as peptidoglycan.

WO2011/029784 describes the anti-inflammatory action of a strain *of Lactobacillus rhamnosus* strain LMG P-25211 on the skin, and use in preventing or treating inflammatory or allergic skin conditions.

WO2010/056198 describes preparations useful for the treatment of Staphylococcus induced infections which comprise a combination of one or more viable α-Streptococcus strains and one or more viable Lactobacillus strains chosen from the groups of *Lactobacillus rhamnosus* strain LB21, *Lactobacillus plantarum* strain LB3, *Lactobacillus plantarum* strain LB7.

WO2006/000992 describes cosmetic treatments intended for preventing and/or treating dry or sensitive skin, where a probiotic microorganism, fraction or metabolite thereof is combined with at least one divalent inorganic cation.

Marsella et al (Veterinary Immunology and Immunopathology 146 (2012) 18 5-189) describe the immunomodulatory effects of oral administration of *Lactobacillus rhamnosus* GG in a model of allergic/atopic dermatitis.

EP2161329A1 describes the immunomodulatory effects of extracts from Lactobacillus bacteria and describes use in the treatment of inflammatory disorders.

Hoang et al (Inflammation & Allergy - Drug Targets, 2010, 9, 192-196) describes the use of orally administered *Lactobacillus rhamnosus* cell lysate as a daily immunobiotic supplement in the treatment of atopic eczema.

WO2011/045471 describes the use of *Lactobacillus rhamnosus* GG in combination with *Lactobacillus rhamnosus* LC705 for use in the treatment of a respiratory infection, in particular for use against viruses causing respiratory infections, e.g. influenza virus.

### Summary of the Invention

The invention provides probiotic bacteria and lysates thereof for use in methods of medical treatment, in methods of cosmetic treatment and for use as an anti-bacterial agent. Use in the treatment of infections, including skin infections and methods involving regeneration or repair of the skin barrier are disclosed.

The invention provides a probiotic bacterium for use in a method of treatment. The probiotic bacterium may be provided in the form of a lysate. In preferred embodiments the probiotic bacterium is a strain of *L.rhamnosus, L.reuteri,* or *Bifidobacterium longum.* Preferably, the probiotic bacterium is *L.rhamnosus* GG. *L.rhamnosus* GG is deposited with ATCC under accession number 53103. The method of treatment may comprise administering *L.rhamnosus* GG to the patient. The method may comprise administering *L.rhamnosus* GG to the skin of the patient. The methods of the invention result in the treatment or alleviation of the condition in the patient undergoing the treatment.

In some embodiments the method of medical treatment is a method of treatment or prevention of infection, for example a bacterial infection such as a Staphylococcus infection. The infection may be a *Staphylococcus aureus* infection. The infection may be a skin infection. The probiotic bacterium, lysate or composition may be administered separately, sequentially or simultaneously with the infective agent, for example to a wound or other breach in the skin barrier.

In some embodiments (or additionally), the method of medical treatment is a method involving repair or regeneration of the skin barrier. In methods involving the repair or regeneration of the skin barrier the probiotic bacterium may be a strain of *L.rhamnosus, L.reuteri* or *Bifidobacterium longum.* The probiotic bacterium may be *L.rhamnosus* GG. In these methods two different strains of probiotic bacterium may be administered in the same method of treatment, for example *L.reuteri* and *L.rhamnosus* GG may be co-administered. The probiotic bacterium may be administered live, inactivated, or as a lysate. Where two or more probiotic bacteria are to be administered, one or more may be administered as a lysate.

Methods of treatment involving repair or regeneration of the skin barrier may involve the repair or regeneration of the skin after injury.

Methods of treatment involving repair or regeneration of the skin barrier may be useful in the treatment of psoriasis, icthyosis, dermatitis, wound healing, acne (including Hiradenitis Suppurativa), Burns, Diaper Rash, Netherton's Syndrome, Actinic Keratosis, Dermatomycoses, Dermatosis or Ectodermal Dysplasia or other disorders associated with damage or breakdown of the skin barrier. Other such disorders will be readily appreciable to the person skilled in the art. Preferably, the methods involve the administration of the probiotic bacterium to the skin.

The invention further provides the use of a probiotic bacterium or lysate thereof in the manufacture of a medicament for the treatment of skin infection and/or repair or regeneration of the skin barrier. The probiotic bacterium may be an *L.rhamnosus, L.reuteri,* or *Bifidobacterium longum* strain. It may be *L.rhamnosus* GG. The medicament may comprise two or more strains of probiotic bacterium, or may be co-administered with another strain of probiotic bacterium or lysate thereof. The medicament may be useful in the repair or regeneration of the skin after injury, or in the treatment of psoriasis, icthyosis, dermatitis, wound healing, acne (including Hiradenitis Suppurativa), Burns, Diaper Rash, Netherton's Syndrome, Actinic Keratosis, Dermatomycoses, Dermatosis or Ectodermal Dysplasia or other disorders associated with damage or breakdown of the skin barrier. Other such disorders will be readily appreciable to the skilled person.

The invention also provides a pharmaceutical composition comprising a probiotic bacterium. The pharmaceutical composition may comprise an *L.rhamnosus* probiotic bacterium such as *L.rhamnosus* GG or *L.reuteri,* or may comprise a *Bifidobacterium longum* probiotic bacterium. The pharmaceutical composition may further comprise another probiotic bacterium strain such as one of *L.reuteri, L.rhamnosus* GG or *Bifidobacterium longum.* The pharmaceutical composition may comprise lysates of one or more of the probiotic bacterial strains, or a mixture of lysates, live or inactivated probiotic bacteria. The pharmaceutical composition may be formulated for administration to the skin of the patient in need of such treatment.

The invention further provides methods of cosmetic treatment involving the administration of probiotic bacterium or lysate thereof to a subject, or use of such probiotic bacterium or lysate thereof in a method of cosmetic treatment. Such embodiments do not involve the treatment of the human or animal body by therapy or surgery. In such cosmetic methods the probiotic bacterium preferably comprises an *L.rhamnosus* probiotic bacterium or lysate thereof, for example *L.rhamnosus* GG, or an *L.reuteri* probiotic bacterium, or lysate thereof, or a *Bifidobacterium longum* probiotic bacterium, or lysate thereof. The methods may involve the administration of the probiotic bacterium to the skin of the subject.

The invention also provides cosmetic compositions comprising an *L.rhamnosus* probiotic such as *L.rhamnosus* GG, *L.reuteri, Bifidobacterium longum* or lysates thereof. The cosmetic composition may comprise one or more of an *L.rhamnosus* probiotic bacterium such as *L.rhamnosus* GG an *L.reuteri,* a *Bifidobacterium longum* or lysates or one, two or each of these probiotic bacteria.

The invention further provides antibacterial compositions comprising a probiotic bacterium or lysate thereof. The probiotic bacterium may be *L.rhamnosus* GG. Such compositions may be useful for killing or inhibiting the action or growth of bacteria. The compositions may be useful for cleaning or pre-treating a surface which has, or is suspected to have, bacteria on it, or is likely to be exposed to bacteria. In some embodiments the antibacterial composition may be formulated so as not to be suitable for administration to a patient or subject.

The invention also relates to methods of preparing compositions, e.g. suitable for therapeutic and/or cosmetic and/or anti-bacterial use. The method may comprise subjecting a population of probiotic bacteria to lysis and formulating the resulting lysate into a composition. Alternatively, the method may comprise formulating intact bacteria into a composition. The method may comprise the step of treating the bacteria or lysate so as to remove or inactivate intact bacteria, for example, by partitioning intact bacteria from the lysate. The lysate may be subject to purification or filtration steps, e.g. to remove intact bacteria, bacterial growth media or contaminants.

The compositions prepared by such a method may be suitable for use in treatment, for example in treating bacterial infections, or for repairing the skin barrier. Such methods may involve the addition of one or more pharmaceutically or cosmetically acceptable excipients. Other compositions may not be suitable for use in treatment, for example antibacterial compositions such as washes.

The following numbered paragraphs (paras.) contain statements of broad combinations of the inventive technical features herein disclosed:-
1. A probiotic bacterium or a lysate thereof for use in a method of treatment, wherein the probiotic bacterium is *Lactobacillus rhamnosus* GG.
2. The probiotic bacterium or lysate thereof for use according to paragraph 1 wherein the method of treatment involves administration of the probiotic bacterium or lysate thereof to the skin of the patient being treated.
3. The probiotic bacterium or lysate thereof for use according to paragraph 1 or paragraph 2 for use in a method of treatment or prevention of infection.
4. The probiotic bacterium or lysate thereof for use according to paragraph 3 wherein the infection comprises a bacterial infection.
5. The probiotic bacterium or lysate thereof for use according to paragraph 4 wherein the bacterial infection is a Staphylococcus infection, preferably an *S.aureus* infection.
6. The probiotic bacterium or lysate thereof for use according to any one of the preceding paragraphs wherein the method of treatment is for a skin infection.
7. A probiotic bacterium or lysate thereof for use in a method of treatment involving repair or regeneration of the skin barrier wherein the probiotic bacterium is an *L.rhamnosus* or *L.reuteri* probiotic bacterium.
8. The probiotic bacterium or lysate thereof for use according to paragraph 7 wherein the method involves administration of the probiotic bacterium or lysate thereof to the skin.
9. The probiotic bacterium or lysate thereof for use according to paragraph 7 wherein the probiotic bacterium is *L.rhamnosus* GG.
10. The probiotic bacterium for use according to any one of paragraphs 7-9 wherein the method is for treatment of psoriasis, icthyosis, dermatitis, wound healing, acne (including Hiradenitis Suppurativa), Burns, Diaper Rash, Netherton's Syndrome, Actinic Keratosis, Dermatomycoses, Dermatosis, or Ectodermal Dysplasia.
11. The probiotic bacterium for use according to any one of the preceding paragraphs wherein the probiotic bacterium is co-administered with a probiotic bacterium of a different strain, or a lysate thereof.
12. The probiotic bacterium or lysate thereof according to any one of the preceding paragraphs wherein the method of treatment involves co-administration with *L.reuteri* or a lysate thereof.
13. A pharmaceutical composition comprising *L.rhamnosus* GG or a lysate thereof.
14. The pharmaceutical composition according to paragraph 13 further comprising a probiotic bacterium of a strain other than *L.rhamnosus,* or a lysate thereof, wherein preferably the probacterium of a strain other than *L.rhamnosus* is *L.reuteri.*
15. A cosmetic method comprising administration of one or more of an *L.rhamnosus* probiotic bacterium such as *L.rhamnosus* GG, *L.reuteri,* or lysates thereof to a subject.
16. The cosmetic method according to paragraph 15 wherein the probiotic bacterium or lysate is administered to the skin of the subject.
17. A cosmetic composition comprising an *L.rhamnosus* probiotic bacterium, or a lysate thereof and/or an *L.reuteri* probacterium, or a lysate thereof.
18. A method of medical treatment comprising administering *L.rhamnosus* GG or a lysate thereof to a patient, thereby treating the patient.
19. The method according to paragraph 18 wherein the probiotic bacterium or lysate thereof is administered to the skin of the patient.
20. The method of medical treatment according to paragraph 18 or paragraph 19 further comprising administering a probiotic bacterium other than *L.rhamnosus* GG, or a lysate thereof, to the patient, wherein the probiotic bacterium other than *L.rhamnosus* is preferably *L.reuteri.*
21. The method of medical treatment according to paragraph 18 wherein the medical treatment is the treatment or prevention of infection.
22. The method of medical treatment according to any one of paragraphs 18 to 21 wherein the medical treatment is the treatment or prevention of skin infection, and wherein the skin infection is treated or alleviated following the treatment.
23. A method of medical treatment comprising administering *L.rhamnosus, L.rhamnosus* GG or *L.reuteri* to a patient in need thereof, wherein the treatment is for the repair or regeneration of the skin barrier, wherein the skin barrier is repaired or regenerated following the treatment.
24. The method of medical treatment according to paragraph 23 wherein the *L.rhamnosus, L.rhamnosus* GG or *L.reuteri* is administered to the skin of the patient.
25. The method according to paragraph 22 or paragraph 23 wherein the method of treatment is for psoriasis, icthyosis, dermatitis, wound healing, acne (including Hiradenitis Suppurativa), Burns, Diaper Rash, Netherton's Syndrome, Actinic Keratosis, Dermatomycoses, Dermatosis, or Ectodermal Dysplasia.
26. Use of a probiotic bacterium or a lysate thereof in the manufacture of a medicament for use in the treatment of infection, wherein the probiotic bacterium is *L.rhamnosus* GG.
27. Use of a probiotic bacterium or a lysate thereof in the manufacture of a medicament for use in treatment, wherein the treatment involves repair or regeneration of the skin barrier, and wherein the probiotic bacterium is an *L.rhamnosus* or *L.reuteri* probiotic bacterium.
28. Use according to paragraph 26 or paragraph 27 wherein the medicament is formulated for administration to skin.
29. Use according to paragraph 28 wherein the method is for treatment of psoriasis, icthyosis, dermatitis, wound healing, acne (including Hiradenitis Suppurativa), Burns, Diaper Rash, Netherton's Syndrome, Actinic Keratosis, Dermatomycoses, Dermatosis, or Ectodermal Dysplasia.
30. Use of a probiotic bacterium or lysate according to any one of paragraphs 26-29 wherein the treatment involves co-administration of more than one probiotic bacterium or lysate thereof.
31. A lysate of *L.rhamnosus* GG.
32. A composition formulated for administration to the skin and comprising *L.rhamnosus* GG, *L.rhamnosus* and/or *L.reuteri,* or a lysate of one or more of said bacteria.
33. An antibacterial composition comprising *L.rhamnosus* GG or a lysate thereof.

Combinations of features of the invention are described in the following paragraphs:
1. A probiotic bacterium or a lysate thereof for use in a method of treatment, wherein the probiotic bacterium is a strain of *Lactobacillus rhamnosus,* a strain of *Lactobacillus reuteri,* or a strain of *Bifidobacterium longum.*
2. A probiotic bacterium or a lysate thereof for use in a method of treatment according to paragraph 1, wherein the method of treatment involves repair or regeneration of the skin barrier.
3. A probiotic bacterium or a lysate thereof for use in a method of treatment according to paragraph 1 or 2, wherein the probiotic bacterium is *Lactobacillus rhamnosus* GG.
4. A probiotic bacterium or a lysate thereof for use in a method of treatment according to any one of paragraphs 1 to 3, wherein the probiotic bacterium is for use in the repair or regeneration of the skin after injury.
5. A probiotic bacterium or a lysate thereof for use in a method of treatment according to any one of paragraphs 1 to 3, wherein the method is for treatment of wound healing, burns, psoriasis, icthyosis, dermatitis, acne (including Hiradenitis Suppurativa), Diaper Rash, Netherton's Syndrome, Actinic Keratosis, Dermatomycoses, Dermatosis, or Ectodermal Dysplasia.
6. A probiotic bacterium or a lysate thereof for use in a method of treatment according to any one of paragraphs 1 to 5, wherein the method of treatment involves administration of the probiotic bacterium or lysate thereof to the skin of the patient being treated.
7. A probiotic bacterium or a lysate thereof for use in a method of treatment according to paragraph 1, wherein the probiotic bacterium is *Lactobacillus rhamnosus* GG and the method of treatment involves the treatment or prevention of infection.
8. A probiotic bacterium or a lysate thereof for use in a method of treatment according to paragraph 7, wherein the infection comprises a bacterial infection.
9. A probiotic bacterium or a lysate thereof for use in a method of treatment according to paragraph 7 or 8, wherein the bacterial infection is a Staphylococcus infection, preferably a *Staphylococcus aureus* infection.
10. A probiotic bacterium or a lysate thereof for use in a method of treatment according to any one of paragraphs 7 to 9, wherein the infection is a skin infection.
11. A probiotic bacterium for use according to any one of paragraphs 1 to 10 wherein the probiotic bacterium is co-administered with a probiotic bacterium of a different strain, or a lysate thereof.
12. A probiotic bacterium or a lysate thereof for use in a method of treatment according to any one of paragraphs 1 to 11 wherein the method of treatment involves co-administration of one of a strain of *Lactobacillus rhamnosus* or a lysate thereof, a strain of *Lactobacillus reuteri* or a lysate thereof, or a strain of *Bifidobacterium longum* or a lysate thereof, with at least another one of said strains or lysates.
13. A cosmetic method comprising administration of a strain of *Lactobacillus rhamnosus* probiotic bacterium or a lysate thereof, and/or a strain of *Lactobacillus reuteri* probiotic bacterium or a lysate thereof, and/or a strain of *Bifidobacterium longum* probiotic bacterium or a lysate thereof, to a subject.
14. A cosmetic method according to paragraph 13, wherein the strain of *Lactobacillus rhamnosus* probiotic bacterium is *Lactobacillus rhamnosus* GG.
15. A cosmetic method according to paragraph 13 or 14, wherein the probiotic bacterium or lysate thereof is administered to the skin of the subject.
16. A cosmetic composition comprising a strain of *Lactobacillus rhamnosus* probiotic bacterium or a lysate thereof, and/or a strain of *Lactobacillus reuteri* probiotic bacterium or a lysate thereof and/or a strain of *Bifidobacterium longum* probiotic bacterium or a lysate thereof.
17. A cosmetic composition according to paragraph 16, wherein the strain of *Lactobacillus rhamnosus* probiotic bacterium is *Lactobacillus rhamnosus* GG.
18. A pharmaceutical composition comprising *Lactobacillus rhamnosus* GG or a lysate thereof.
19. The pharmaceutical composition according to paragraph 18 further comprising a probiotic bacterium of a strain other than *Lactobacillus rhamnosus,* or a lysate thereof, wherein preferably the probiotic bacterium of a strain other than Lactobacillus rhamnosus is a strain of *Lactobacillus reuteri.*
20. A lysate of *Lactobacillus rhamnosus* GG.
21. A composition formulated for administration to the skin and comprising *Lactobacillus rhamnosus* GG, and/or a strain of *Lactobacillus rhamnosus* and/or a strain of *Lactobacillus reuteri,* and/or a strain of *Bifidobacterium longum* or a lysate of one or more of said bacteria.
22. An antibacterial composition comprising *Lactobacillus rhamnosus* GG or a lysate thereof.

### Description of Preferred Embodiments

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art. All documents mentioned in this text are incorporated herein by reference.

Skin is the primary barrier between the organism and the outside world. It must both prevent invasion by pathogens but at the same time, prevent water and electrolyte loss from the body. To date, the barrier function of skin has thought to be solely the role of the stratum corneum. However, complexes known as tight junctions are also critical to the skins' barrier function. Occasionally, the barrier of skin is breached due to wounds or abrasions. In these instances, the skin must repair itself. However, occasionally, repair does not occur quickly enough and this leaves the skin open to infection. We have screened a range of probiotic bacteria for their ability to:
1) Protect skin from the harmful pathogen, *Staphyloccus Aureus* (including methicillin resistant strains)
2) Stimulate regeneration and improve the barrier function of skin cells.

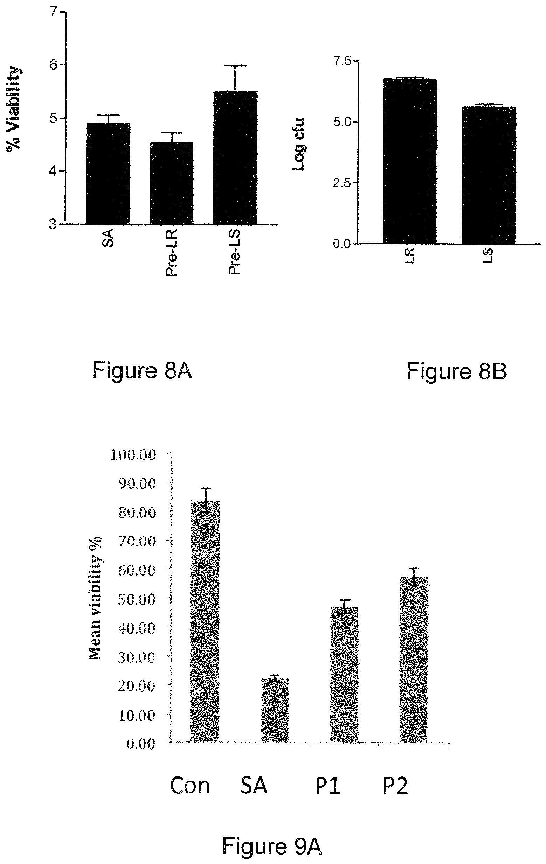

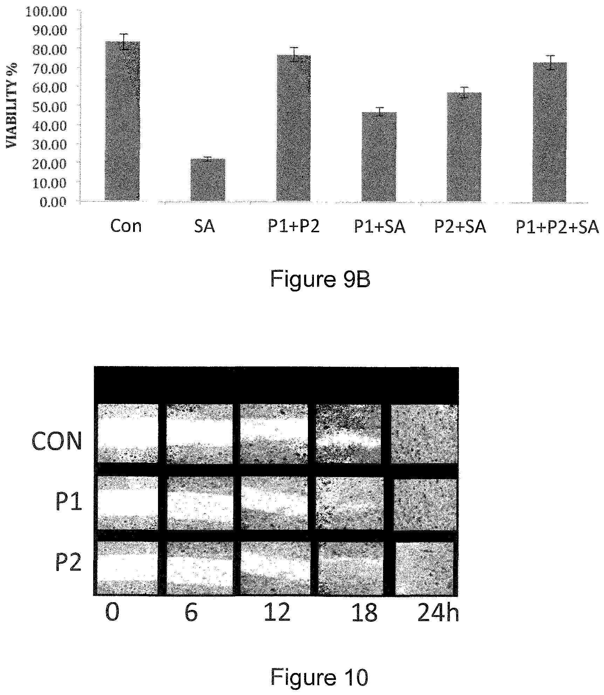

We have identified a strain of probiotic bacterium *(L.rhamnosus* GG) with skin cell protecting and regenerative properties. Significantly, lysates of this strain improve skin keratinocytes ability to resist harmful toxicity induced by S. *aureus* (SA) (Figure 9A). When used in combination with a second strain lysate (*L.reuteri*)*,* the combination is even more effective at protecting against S aureus than when either strain is used alone (Figure 9B). We have also demonstrated that when keratinocyte monolayers are scratched to remove cells (i.e. a model wound), the presence of our propriety probiotic lysates significantly and surprisingly speeds up the recovery of the monolayer and re-establishment of the barrier (Figure 10).

Agents that increase and repair the barrier function of keratinocytes, have great potential not only as therapeutic agents but also as cosmetic skin improving agents. Augmentation of the barrier function increases the skins hydration level and is known to improve the appearance of the skin and lead to a healthier cosmetic appearance.

Our observations broaden the utility and application of probiotics. Our initial observations indicate that probiotics could be applied to a number of applications in skin care that include: wound healing, coating technology for wound dressings, improved skin barrier creams for cosmetic and therapeutic uses (e.g. atopic dermatitis); antibacterial products such as hand washes and soaps, and cosmetic preparations aimed at maintenance/repair of the skin's barrier function and treatments to restore a damaged barrier due to sun exposure and ageing.

With the potential to prevent the detrimental effects of harmful bacteria and simultaneously actively repair a damaged skin barrier the commercial applications for probiotics in skin care are significant. Increasingly people are demanding natural, safe and effective treatments for common skin alignments and our technology represents a significant advance in meeting these market needs

Our technology could not only provide a new therapeutic agent for establishment/repair of the skin barrier, but could potentially circumvent the need for existing aggressive therapies e.g. antibiotics. Current mainstay prevention methods include antibacterial agents that although effective have a number of detrimental effects. Treatment relies on the use of antibiotic to kill harmful bacterial infections. Our approach not only inhibits harmful skin pathogens, but actively promotes repair of the barrier at a cellular level. Since a decline in barrier function/repair goes hand in hand with reduced ability to repel infection, our lysate represents a technology that enhances both the barrier and its ability to guard against pathogens.

There are currently very few products on the market with live bacteria as components (e.g. Nude live skin probiotic cosmetic range). Our purified lysate approach has a number of functional and commercial benefits over these approaches by offering a controllable and definable activity at lower concentrations; increased stability and ease of production; and an improved shelf life. Taken together these make our technology more amenable to therapeutic and cosmetic uses, unlike difficult to manufacture and distribute live bacterial creams.

### Brief Description of the Figures

Embodiments and experiments illustrating the principles of the invention will now be discussed with reference to the accompanying figures in which:

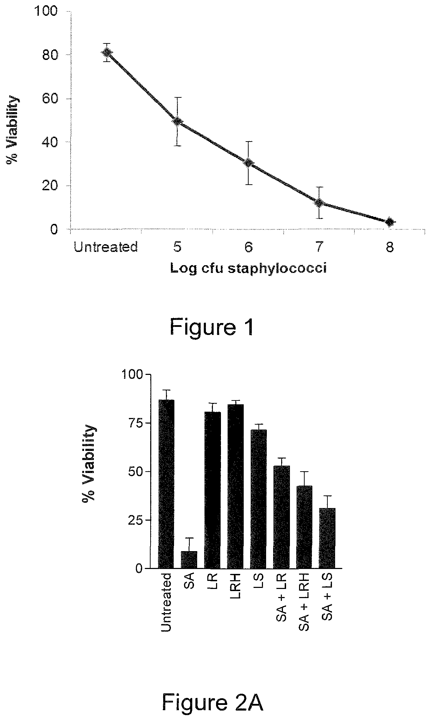

**Figure 1****.** Chart showing S. *aureus* has dose dependent effects on keratinocyte viability. Uninfected cells had a viability of 81.2 ± 4.1%. Cells exposed to 10⁵cfu/ml S. *aureus* had a viability of 49.4 ± 11.1%. Cells exposed to 10⁶ cfu/ml S. *aureus* had a viability of 30.5 ± 9.8 %. Cells exposed to 10⁷cfu/ml S. *aureus* had a viability of 12.1 ± 1.1% while cells exposed to 10⁸cfu/ml S. *aureus* had a viability of 3.3 ± 1.1%. Linear regression analysis confirmed a linear relationship between concentration and percentage viability (p < 0.001). Results are expressed as mean ± SEM.

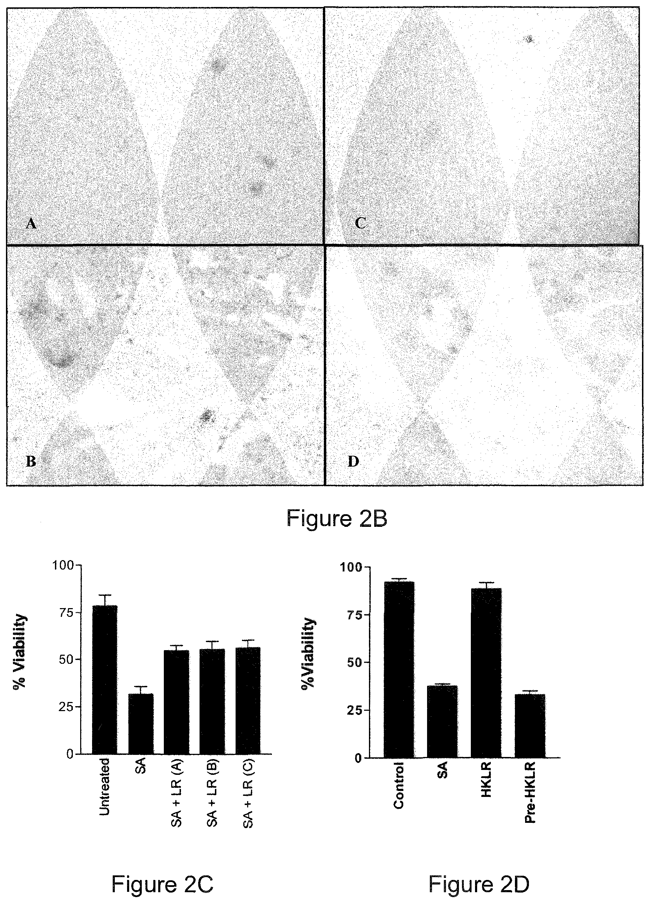

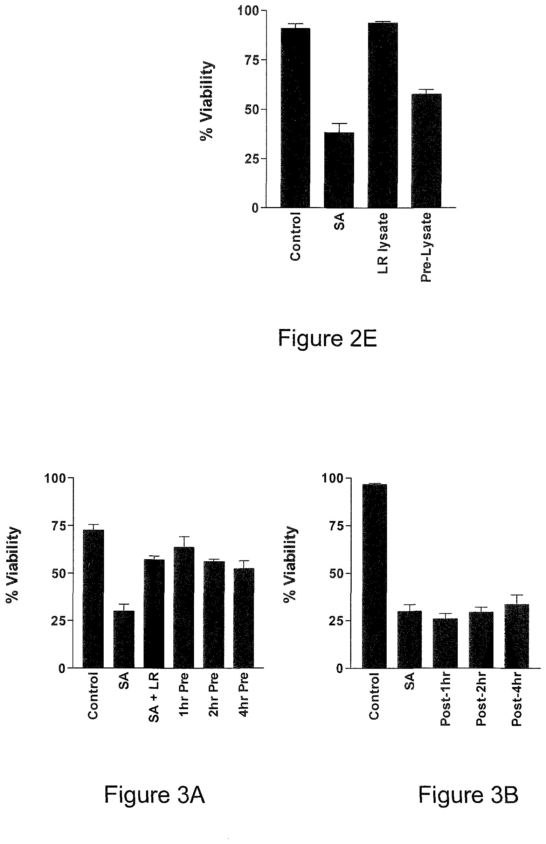

**Figure 2****.** Lactobacilli protect keratinocytes from the cytotoxic effects of S. *aureus.* (A) Chart showing percentage viability for uninfected NHEK (86.9% ± 5.1) and NHEK infected with 10⁸ cfu/ml of S. *aureus* (SA),(8.8 % ± 7.1) *L. reuteri* (LR), (80.8 % ± 4.5) *L*. *rhamnosus* (LRH),(84.8 % ± 2.1) *L. salivarius* (LS), (71.7 % ± 2.9) S. *aureus* & *L. reuteri* (SA+LS), (53.1 % ± 4.2) S. *aureus* & *L. rhamnosus* (SA+LRH), (42.7 % ± 7.4) and S. *aureus* & *L. salivarius* (SA+LS) (31.1% ± 6.5). **(B)** Representative images of infected cells stained with trypan blue (Magnification x 200). A) No treatment, B) infected with 10⁸cfu/ml S. *aureus,* C) exposed to 10⁸cfu/ml *L. reuteri,* or D) infected with 10⁸cfu/ml S. *aureus* and 10⁸cfu/ml *L. reuteri* simultaneously. (C) Chart showing viability of cells infected with either 10⁶ S. *aureus* (SA) (31.6 ± 4.1%), or S. *aureus* and 10⁶ *L. reuteri* (A) (54.8 ± 2.7%), S. *aureus* and 10⁷ *L. reuteri* (B) (55.4 ± 4.3%) and S. *aureus* and 10⁸ *L. reuteri* (C) (56.2 ± 4.1%) (D) Chart showing keratinocytes exposed to S. *aureus* (SA) had reduced viabilities of 37.4 ± 1.3 % compared to controls (92 ± 1.9 %) while keratinocytes exposed to heat killed *L. reuteri* (HKLR) had little change to their viability (88.3 ± 3.3 %). Keratinocytes pre-exposed to heat killed *L. reuteri* (Pre-HKLR) had similar viabilities to S. *aureus* exposed keratinocytes (33 ± 2.1 %). (E) Chart showing *L. reuteri* lysate (LR Lysate) was able to protect keratinocytes, P=0.01. Cells exposed to S. *aureus* (SA) alone had significantly lower viability (38.3 ± 4.7 log cfu) than cells pre-exposed to a lysate of *L. reuteri* (Pre-Lysate)(57.7 ± 2.4 log cfu). Results are expressed as mean ± SEM.
**Figure 3****.** *L. reuteri* protects keratinocytes only if added prior to infection with S. *aureus.* (A) Chart showing percentage viability was significantly higher in cells that were co-infected (SA + LR) (57.1 ± 2, P=0.0016) and pre-exposed for 1 (1hr Pre) (63.6 ± 5.6%, P=0.0003), 2 (2hr Pre) (56.1 ± 1.2%, P=0.0022) and 4 hrs (4hr Pre) with *L. reuteri* (52.4 ± 4.1%, P=0.0066) compared to *S*. *aureus* (SA) infected cells (29.8 ± 3.8). **(B)** Chart showing there was no significant difference between the viability of cells treated with *L. reuteri* 1 (Post-1hr) (25.8 ± 0.6%), 2 (Post-2hr) (29.3 ± 2.6%), or 4 hours (Post-4hr) (33.57 ± 4.9%) after infection had begun and cells that had been infected with *S*. *aureus* (SA) alone (29.9± 3.4%) (P>0.05). Results are expressed as the mean ± SEM.

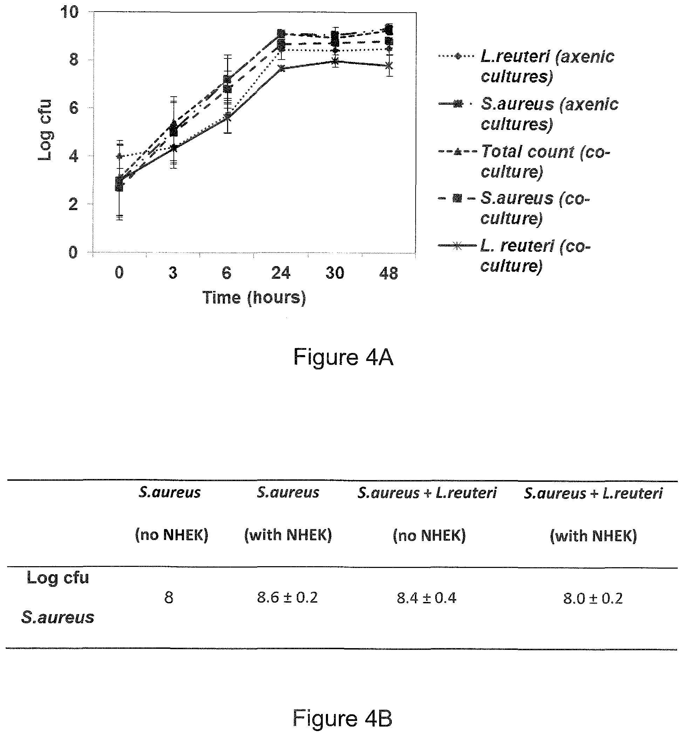

**Figure 4a****.** *L. reuteri* does not inhibit the growth of S. *aureus* in co-culture. Chart showing results of competition assay revealing no significant difference between groups over time (P=0.146).
**Figure 4b** **(Table 1).** *L. reuteri* does not affect staphylococcal viability in cell culture. *S. aureus* grown in the absence of cells had a viable count of 8.0 (log cfu). *S. aureus* grown in the presence of keratinocytes had a viable count of 8.6 ± 0.2. *S. aureus* co-incubated with *L. reuteri* in the absence of cells had a viable count of 8.4 ± 0.4 while *S. aureus* co-incubated with *L. reuteri* in the presence of cells had a viable count of 8.0 ±0.2. There was no significant difference found between groups (P=0.34). Results are expressed as the mean ± SEM.

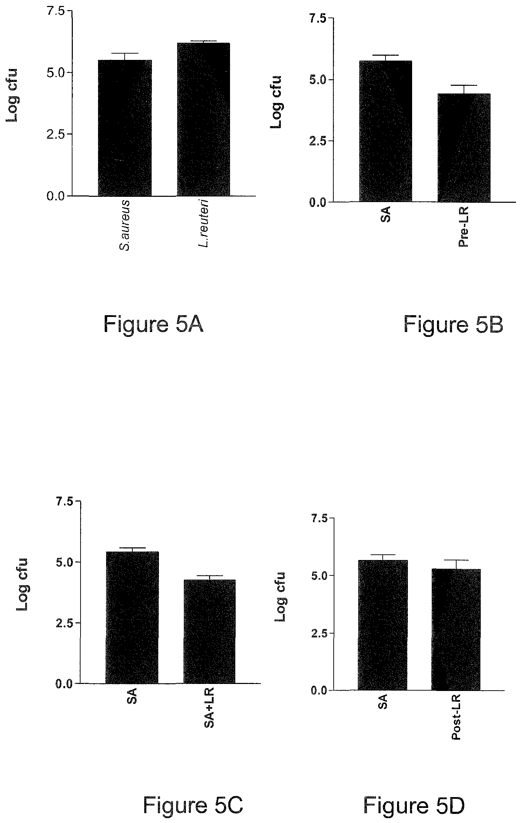

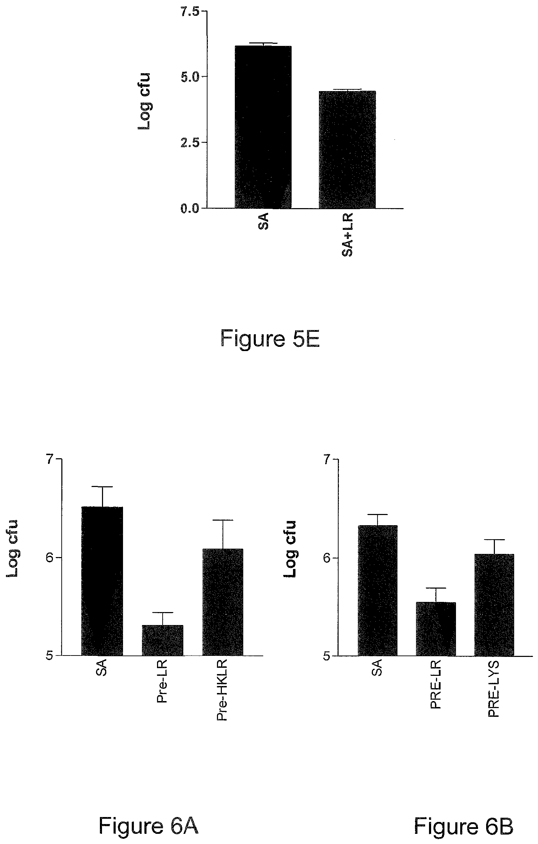

**Figure 5****.** *L. reuteri* inhibits staphylococcal adhesion and invasion of keratinocytes. Charts showing: (A) *S. aureus* adheres at approximately 5.5 ± 0.3 log cfu/ml and *L. reuteri* adheres at approximately 6.2 ± 0.1 log cfu/ml. (B) Exclusion. Cells pre-exposed to *L. reuteri* before *S. aureus* (Pre-LR) infection had significantly less staphylococci adhered to them (5.7 ± 0.2 log cfu) compared to cells infected with *S. aureus* (SA) alone (4.4 ± 0.4 log cfu). (C) Competition. Cells co-infected with *L. reuteri* (SA+LR) had significantly less staphylococci adhered to them (4.4 ± 0.4 log cfu) compared to cells infected with *S. aureus* (SA) alone (5.6 ± 0.1 log cfu). (D) Displacement. There was no significant difference in the number of adherent staphylococci on cells exposed to *L. reuteri* after *S. aureus* infection had begun (Post-LR) (5.3 ± 0.5 log cfu), compared to cells infected with S. *aureus* (SA) alone(5.7 ± 0.3 log cfu, P=0.47). (E) Cells co-infected with *L. reuteri* had significantly fewer internalised staphylococci (4.5 ± 0.1 log cfu) than cells infected with S. *aureus* alone (6.1 ± 0.1 log cfu). Results are expressed as the mean ± SEM.
**Figure 6****.** Heat killed *L. reuteri* was not able to inhibit staphylococcal adhesion to keratinocytes, but lysates could. Charts showing: (A) The number of staphylococci adherent to NHEK when applied alone (SA) (6.5 ± 0.2 log cfu), when added after NHEK were pre-exposed to live *L. reuteri* (Pre-LR) (5.3 ± 0.1 log cfu), and when pre-exposed to heat killed *L. reuteri (Pre-HKLR)* (6.1± 0.3 log cfu). Cells exposed to untreated *L. reuteri* and had significantly fewer staphylococci bound to them (P=0.003), but cells exposed to heat killed *L. reuteri* had no significant difference in adherent staphylococci (P=0.09). (B) The number of staphylococci adherent to NHEK when applied alone (SA) (6.3 ± 0.1 log cfu), was significantly more than when added after NHEK were pre-exposed to live *L. reuteri* (PRE-LR) (5.6 ± 0.2 log cfu, P=0.0002), and when pre-exposed to lysates of *L. reuteri* (PRE-LYS) (6.0± 0.2 log cfu, P=0.032). Results are expressed as the mean ± SEM.

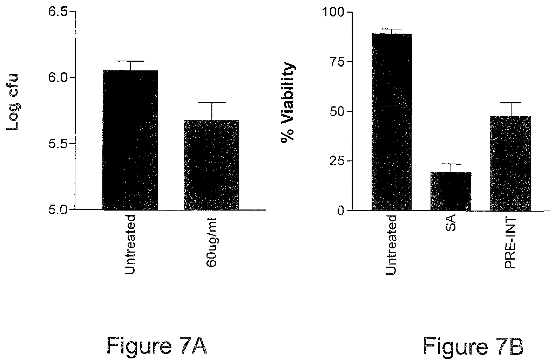

**Figure 7****.** *S. aureus* utilises α5β1 integrin to bind to keratinocytes and blocking of this integrin is sufficient to protect keratinocytes from the effects of *S.aureus.* Charts showing: (A) Significantly fewer staphylococci adhered to cells treated with 60µg/ml blocking antibody (5.7 ± 0.1 log cfu) compared to untreated keratinocytes (6.1 ± 0.1 log cfu, P=0.007). Results are expressed as the mean ± SEM. (B) Significantly more keratinocytes were viable after infection with *S.aureus* for 24 hours if pre-exposed to an anti- α5β1 integrin (PRE-INT) antibody prior to infection (47.9 ± 6.0 %) compared to keratinocytes infected with *S.aureus* alone (SA) (19.3 ± 2.0 %, P=0.03). Results are expressed as the mean ± SEM.
**Figure 8****.** *L. salivarius* was not able to inhibit staphylococcal adhesion to keratinocytes. Charts showing: (A) Keratinocytes pre-exposed to *L. salivarius* (PRE-LS) had a similar number of adherent staphylococci (4.5 ± 0.2 log cfu) compared to cells exposed to *S.aureus* alone (SA) (4.9 ± 0.2 log cfu). (B) *L. reuteri* (LR) adhered significantly better (6.7 ± 0.1 log cfu) than *L. salivarius* (LS) (5.6 ± 0.1 log cfu, P=0.005). Results are expressed as mean ± SEM.
**Figure 9****.** Charts showing: (A) Human primary keratinocytes exposed to the pathogen S. *aureus* (SA) for 24 hours. This results in approximately 80% of the cells dying with only 20% of the keratinocytes viable after 24 hours. In the presence of lysates P1 (LGG) or P2 (*L.reuteri*)*,* a significantly higher number of the keratinocytes remain viable after 24 hours exposure to pathogen (50% and 60% respectively), (B) Probiotic lysates P1 (LGG) and P2 (*L.reuteri*) are more efficacious when applied simultaneously and have a greater protective effect on the viability of the keratinocytes than when each strain is applied separately.
**Figure 10****.** Images from 'Wounding' scratch assays. In 'Wounding' scratch assays both P1 (LGG) and P2 (*L.reuteri*) lysates speed up barrier repair. At 18 hours post scratching, the cells in lysate treated samples (P1 and P2) have almost repaired the damage caused by the scratch whereas in untreated cells (con) the scratch is still apparent.

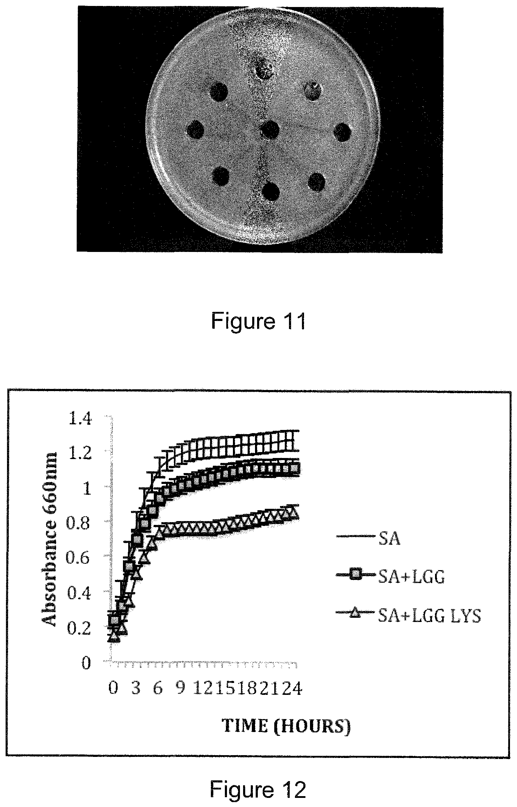

**Figure 11****.** Image of well difusion assay. Well diffusion assay showing that AC413 does not inhibit the growth of *S. aureus.* For this experiment, *S aureus* was plated as a lawn of bacteria. Wells were then dug into the agar and AC413 placed in the wells. Any killing of *S. aureus* by AC413 would be seen as a zone of inhibtion around the well.
**Figure 12****.** Chart showing LGG reduces the growth rate of *S. aureus.* In this experiment, *S. aureus* (SA) and LGG were grown together (SA+LGG). An extra experiment also involved the growth of *S. aureus* with an LGG lysate (SA+LGG LYS). The graphs demonstrate that the growth of *S. aureus* is slower in the presence of either live LGG or the lysate.

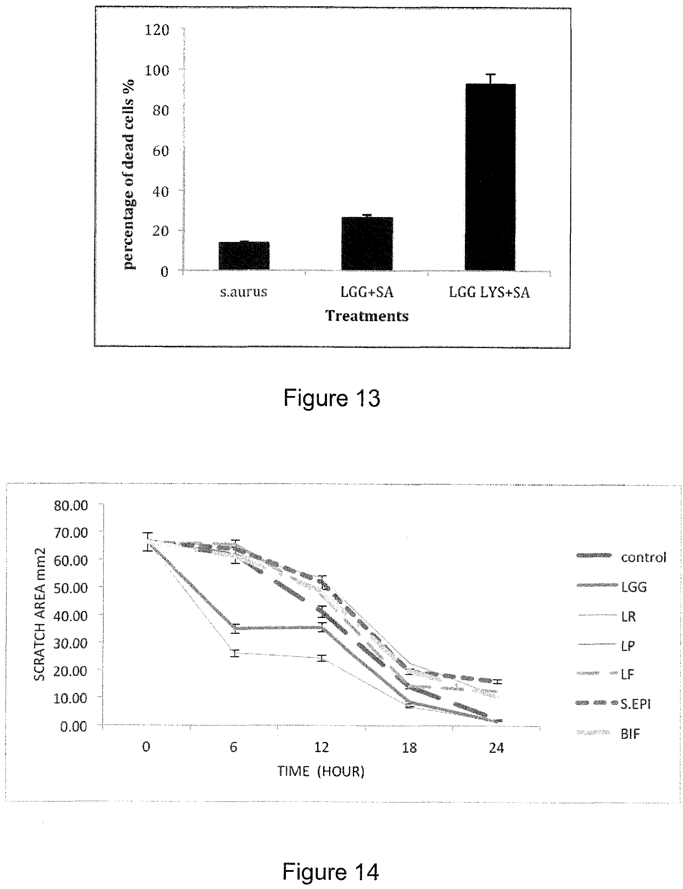

**Figure 13****.** Chart showing LGG and its lysate kill *S. aureus.* In this experiment the number of dead *S. aureus* were measured in the presence of live LGG or an LGG lysate. The highest number of dead bacteria are seen with the LGG lysate.
**Figure 14****.** Chart showing LGG or *L reuteri* lysates increase the rate of re-epithelialisation of keratinocyte monolayers. In this experiment, a keratinocyte monolayer was scratched and the rate of re-epithelialisation measured in the presence of either LGG, *L reuteri* (LR), *L.plantarum* (LP), *L.fermentum* (LF) *S.epidermidis* (S EPI) or *Bifidobacterium longum* (BIF). Only LGG or *L reuteri* increase the rate of re-epithelialisation.

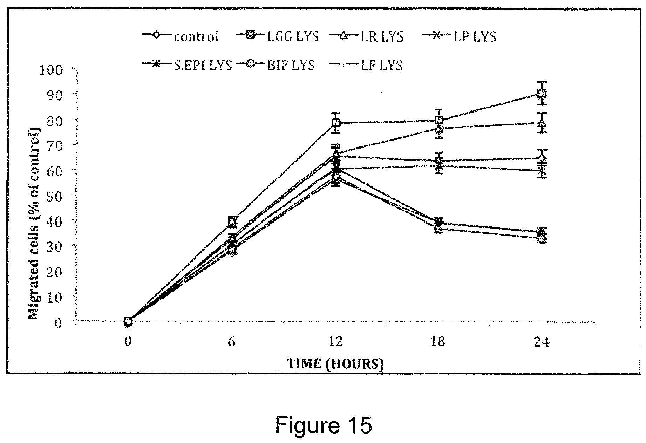

**Figure 15****.** Chart showing LGG and *L.reuteri* increase the rate of keratinocyte migration. In this experiment, the migration of keratinocytes was measured because this is an important mechanism by which monolayers re-epithelialise. Only LGG or *L.reuteri* increase migration of keratinocytes.

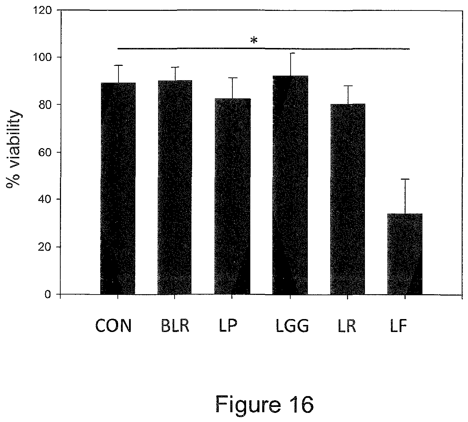

**Figure 16****.** Chart showing probiotic bacteria have strain-dependent effects on keratinocyte viability. Human primary epidermal keratinocytes were incubated with 10⁸ CFU/ml bacteria for 24 hours. Following exposure, the viability of keratinocytes was measured using a MTT assay. The viability of keratinocytes incubated in the presence of *B. longum reuter* (BLR), *L. plantarum* (LP), *L. reuteri* (LR) or *L. rhamnosus* Goldwin and Gorbach (LGG), was not significantly different to that of untreated cells (CON). However, keratinocyte cultures treated with *L. fermentum* (LF) had reduced viability compared to control (∼50% reduction in viability, p<0.005).

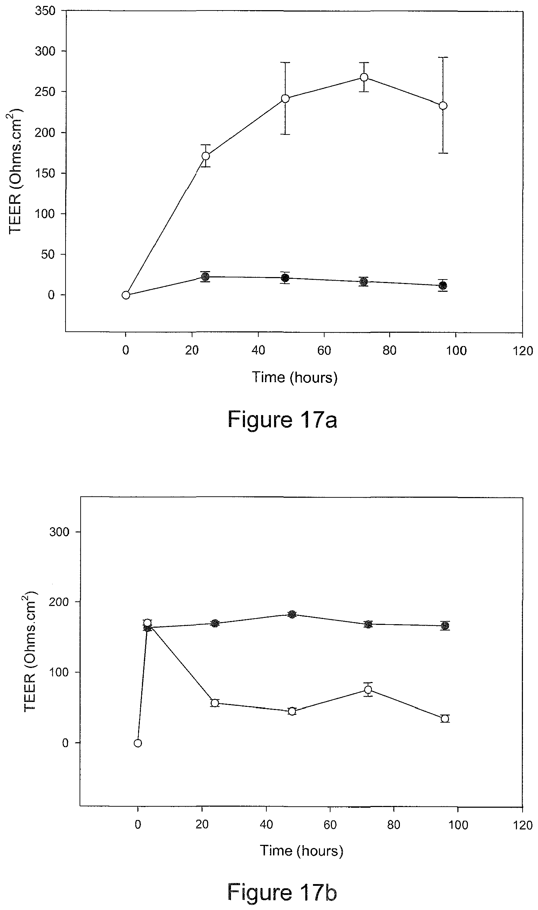

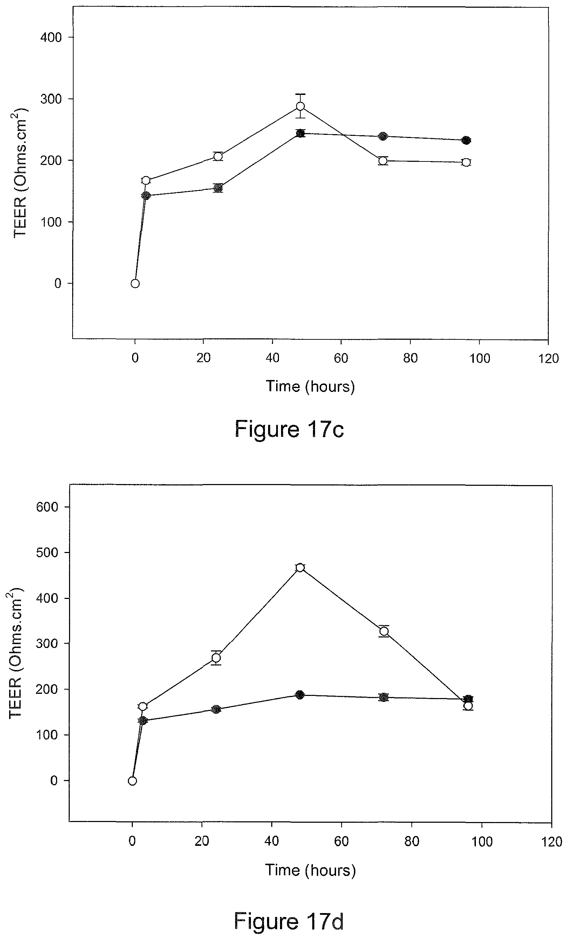

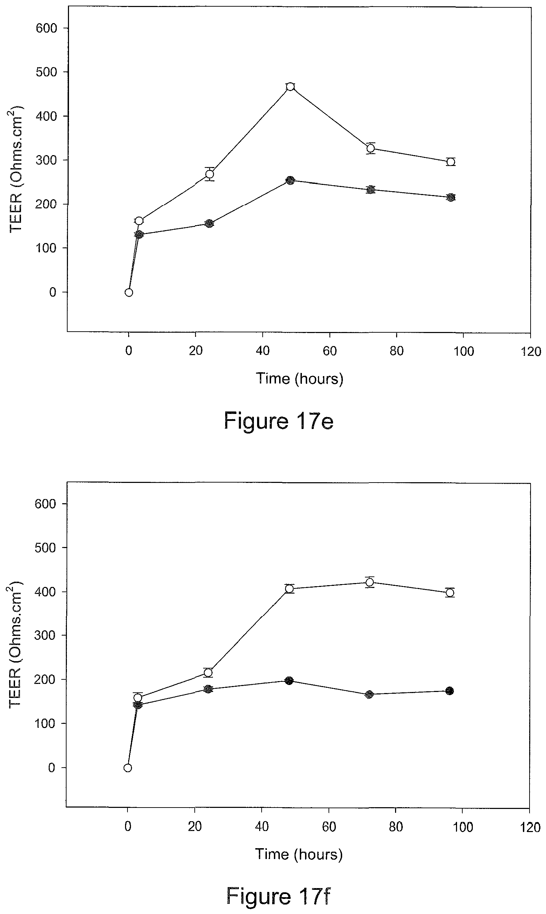

**Figure 17****.** Charts showing probiotic bacteria enhance tight junction barrier function with strain specific effects. Human primary keratinocytes were induced to form TJs and the TEER of the monolayers was monitored with time in untreated monolayers vs monolayers infected with probiotic bacteria. In control monolayers, TEER developed with time post calcium switch and reached a peak of around 255 Ohms.cm² +/- 50.4. With the exception of L. fermentum (which decreased the TEER relative to control monolayers) all the probiotic bacteria increased TEER over control levels in a strain dependent manner. *L. rhamnosus* GG and B. *longum reuter* produced the greatest and most sustained effects. In Figure 17, open circles are treated monolayers and closed circles are untreated monolayers 17(a) development of TEER in untreated cells in response to increasing calcium concentration. (b) effect of *L. fermentum* (c) *L. reuteri* (d) *L. plantarum* (e) *B. longum reuter* (f) *L. rhamnosus* GG, on development of TEER compared to untreated control.

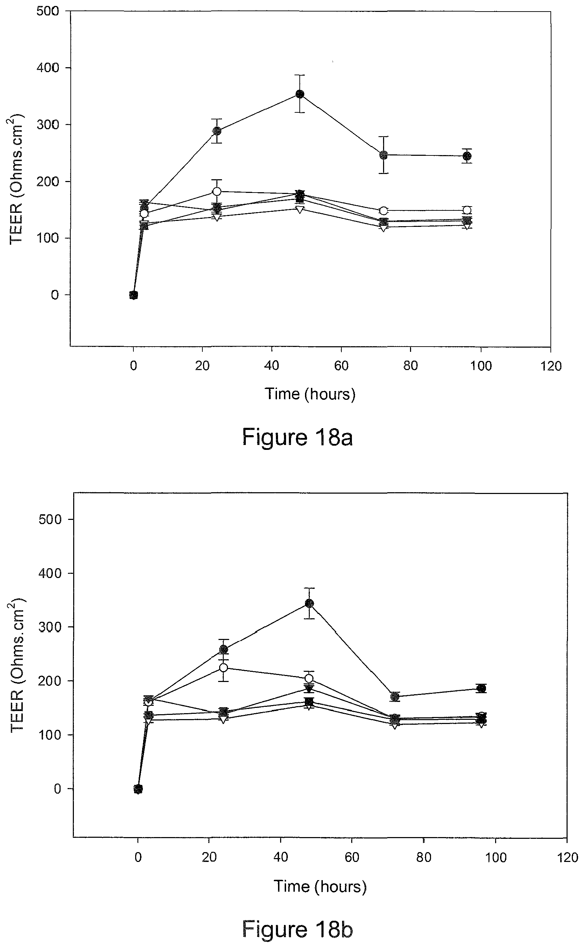

**Figure 18****.** Charts showing probiotic bacteria have dose-dependent effect on TEER in human keratinocytes. Human keratinocytes were treated with lysates made from *B*. *longum reuter* (BLR) or *L. rhamnosus* GG (LGG) at concentrations of 10⁸ 10⁶, 10⁴ and 10² CFU/ml and the effects on TEER measured with time. *B. longum reuterwas* only effective at a concentration of 10⁸ CFU/ml. However, *L rhamnosus* GG was also effective at 10⁶ and 10⁴ CFU/ml. (a) effects on TEER of *B. longum reuter* (b) effects on TEER of *L. rhamnosus* GG. For 18(a) and (b) Δ is control, untreated, ■ is effect of 10² bacteria, ▲ is effect of 10⁴ bacteria, O is effect of 10⁶ bacteria, ● is effect of 10⁸ bacteria.

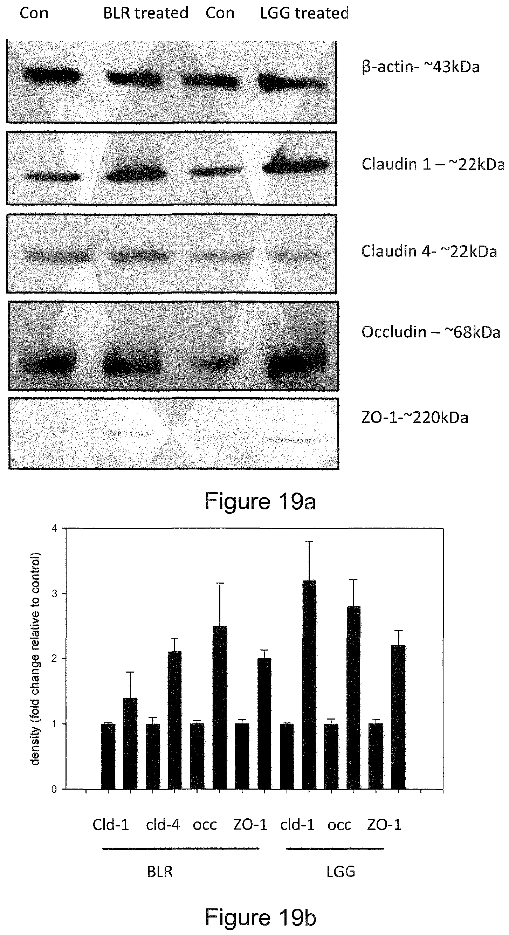

**Figure 19****.** Immunoblot and chart showing probiotic bacteria modulate tight junction protein expression in human keratinocytes. Human keratinocytes were treated with lysates from either 10⁸ CFU/ml *L. rhamnosus* GG (LGG) or *B. longum reuter* (BLR) for 24 h. Subsequently, the keratinocytes were harvested and the expression of claudin 1, claudin 4, ZO-1 and occludin was investigated using immunoblotting (a) and subsequent densitometry (b). BLR increased the expression of all four TJ proteins relative to the control (claudin 1 (cld-1) 3.7X +/- 0.08 (p<0.05), Claudin 4 (cld-4) -2.15 +/- 0.02 (p<0.05), occludin (occ), 2.53X +/- 0.14 (P<0.005), ZO-1, 2X+/- 0.024 (p<0.05). However, LGG affected no change to claudin 4 levels but increased the expression of the other three proteins (claudin 1 - 3.27x +/-0.36 (p<0.05), occludin 2.65x +/- 0.17 (p<0.005), ZO-1 - 2.22x +/- 0.036 (p<0.05).

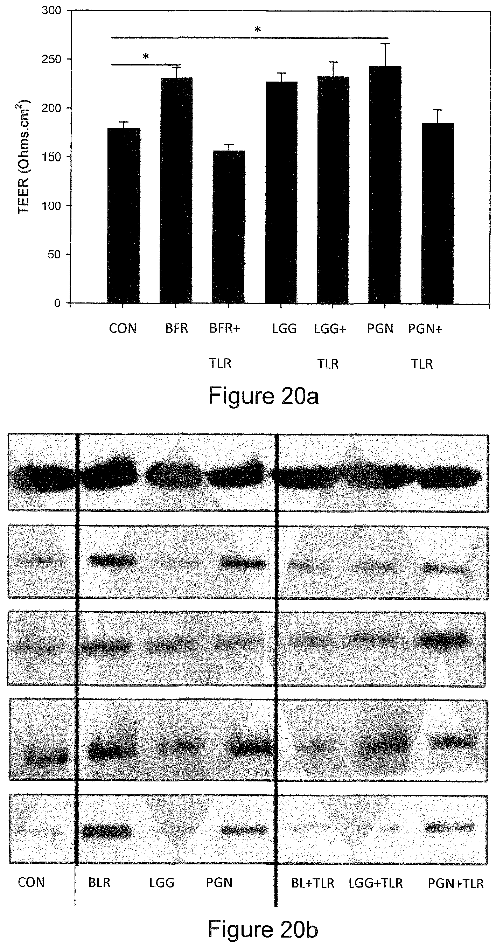

**Figure 20****.** Immunoblot and chart showing neutralisation of TLR2 abolishes specific probiotic mediated effects on TJ barrier function and protein expression. Keratinocytes were treated with a TLR2 neutralising antibody prior to incubation with lysates from B. *longum reuter* (BLR) or *L. rhamnosus* GG (LGG). (A) Chart showing that in cells treated with BLR but not LGG, the probiotic-induced increase in TEER was abolished by incubation with the antibody. (B) Immunoblot similarly showing the increase in TJ protein expression was also abolished by neutralisation of TLR 2 in BLR, but not LGG-treated cells.

### Detailed Description of the Invention

The details of one or more embodiments of the invention are set forth in the accompanying description below including specific details of the best mode contemplated by the inventors for carrying out the invention, by way of example. It will be apparent to one skilled in the art that the present invention may be practiced without limitation to these specific details.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

### Probiotic Bacteria

The invention relates to the use of probiotic bacteria. Probiotics are commonly defined as "live microorganisms which when administered in adequate amounts confer a health benefit on the host". Studies in the gut have demonstrated the ability of probiotic bacteria to inhibit colonisation by pathogens through mechanisms including exclusion, competition and displacement of pathogen attachment to the host tissues. As used herein, the term "probiotic bacterium" may also refer to such bacteria when they are no longer alive, for example following inactivation by heat or radiation.

### Lactobacillus rhamnosus

The invention particularly relates to probiotic bacteria of the species *Lactobacillus rhamnosus.* Such bacteria were originally considered a subspecies of *Lactobacillus casei,* but later genetic research found it to be a species of its own. A number of *L.rhamnosus* strains are known. For example strains 1-1720 (Pasteur Collection Nationale de Cultures de Microorganismes), AC413, GR-1 (Karlsson et al., BMC microbiology 2012, 12:15), JB-1 (Bravo et al., PNAS 2011, 108(38) 16050-16055), GG and Lc705 (Savijok et al., J. Proteome Res. 2011 10(8): 3460-3473). Other strains of *L.rhamnosus* may be readily isolated. Optionally, some embodiments of the present invention do not include the use of *Lactobacillus rhamnosus* strain LB21.

In particular, the invention relates to *L.rhamnosus* GG. *L.rhamnosus* GG (also referred to herein as LGG) is deposited at ATCC (American Tissue Culture Collection) under accession number ATCC 53103. *L.rhamnosus* GG was isolated in 1983 from the intestinal tract of a healthy human being by Gorbach and Goldin,

### Lactobacillus reuteri

The invention also relates to *Lactobacillus reuteri* strains. *L.reuteri* is a gram-positive bacterium that naturally inhabits the gut of mammals and birds. A number of *L.reuteri* strains are known. For example, DSM 17938 and ATCC deposits 23272, 53608, 53609 and 55148 and 55739. *L.reuteri* strains are described in, for example, US6,872,565, and US7,517,681. Other strains of *L.reuteri* may be readily isolated.

A particularly preferred *L.reuteri* strain according to the invention is deposited at ATCC under accession number ATCC 55730, described in US 5,837,238.

### Bifidobacterium longum

The invention also relates to *Bifidobacterium longum* strains. *Bifidobacterium longum* is a species of gram-positive bacterium found in the intestines of infant humans. A number of *Bifidobacterium longum* strains are known. For example, ATCC deposits 15708, 55816, 55818, 15707, 35183, and 51870. Other strains of *Bifidobacterium longum* may be readily isolated.

A particularly preferred *Bifidobacterium longum* strain according to the invention is *Bifidobacterium longum reuter.* In some embodiments the *Bifidobacterium longum reuter* is deposited at ATCC under accession number ATCC 51870.

### Applications

Probiotic bacteria or their lysates according to the invention may be formulated as pharmaceutical compositions for clinical use and may comprise a pharmaceutically acceptable carrier, diluent or adjuvant. They may be formulated for topical administration.

Administration is preferably in a "therapeutically effective amount", this being an amount sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins. It will be appreciated by one of skill in the art that appropriate dosages of the active compounds, and compositions comprising the active compounds, can vary from patient to patient.

### Therapeutic applications

The compounds and compositions of the present invention are useful in the treatment of a wide range of diseases and conditions. In particular, the compounds and compositions of the present invention are useful in the treatment of disorders, diseases and conditions of the skin and find application in the treatment of fibrosis (including post-surgical fibrosis), tissue adhesion formation, and scar formation. The compounds and compositions of the invention are useful in the treatment of acne, eczema, ichthyosis and psoriasis. The probiotic bacteria and their lysates according to the invention are useful in wound healing.

Probiotic bacteria and lysates thereof for use in therapeutic applications may be formulated as medicaments, that is to say formulated as a medicine. The medicament may include other pharmaceutically acceptable ingredients well known to those skilled in the art, including, but not limited to, pharmaceutically acceptable carriers, adjuvants, excipients, diluents, fillers, buffers, preservatives, anti-oxidants, lubricants, stabilisers, solubilisers, surfactants (e.g., wetting agents), masking agents, colouring agents, flavouring agents, and sweetening agents. The formulation may further comprise other active agents, for example, other therapeutic or prophylactic agents.

### Infections

The invention relates to the treatment of infections. The probiotic bacteria and lysates of the invention exhibit anti-infective agent activity. For example, anti-bacterial activity. Thus, they are useful for the treatment of infections including bacterial infections. Thus, they are useful in the treatment of multi-drug resistant bacterial infections, hospital acquired bacterial infections, antibiotic resistant bacterial infections, infections by gram-negative and/or gram -positive bacterial infections.

The probiotic bacteria and lysates of the invention are also useful for the treatment of infections by *Staphylococcus spp., Pseudomonas spp., Staphyloccus saprophyticus, Staphyloccocus xylosus, Staphyloccocus lugdunensis, Staphyloccocus schleiferi, Stapylococcus caprae staphylococcus epidermidis, Staphylococcus saprophyticus, Staphylococcus warneri, Staphylococcus aureus, Staphylococcus hominis,* Methicillin-resistant *Staphylococcus aureus* (MRSA), *Enterococcus faecalis,* Vancomycin-resistant *Enterococcus* (VRE), *Proprionibacterium acnes, Bacillus cereus, Bacillus subtilis, Listeria monocytogenes, Streptococcus pyrogenes, Streptococcus salivariu, Streptococcus mutans* and *Streptococcus pneumonia.*

In particular, the probiotic bacteria and lysates of the invention exhibit anti-*Staphylococcus* activity, and are thus useful for the treatment or prevention of *Staphylococcus* infection. For example, the probiotic bacteria and lysates of the invention exhibit *anti-Staphylococcus aureus* activity, and are thus useful in the treatment or prevention or *Staphylococcus aureus* infections.

Infections occur where disease causing microorganisms invade the tissues of the body. Multiplication of those microorganisms and the toxins that they produce react with the tissues of the body, often causing immune reactions by the infected host. Infections may be caused by bacteria, viruses, viroids, fungi and other parasites. Infections may occur via any of the tissues of the body, such as the skin, gut or membranes. In some embodiments of the invention the probiotic bacteria or lysates of the invention are used to treat infection of tissues other than the gut, for example in some embodiments the probiotic bacterium or lysate according to the invention is not used for the treatment of infection of the alimentary canal, esophagus, stomach, intestines, rectum or anus. In particular aspects the invention relates to the treatment or prevention of infection of the external surface of the body, and particularly of the skin.

The probiotic bacterium, lysates and compositions according to the invention may be used in the treatment or prevention of skin infections. The infection may be due to a bacterium, such as Staphylococcus bacteria. The infective agent may be *Staphylococcus aureus.* The probiotic bacterium, lysate or composition may be applied separately, sequentially or simultaneously with exposure to the infective agent. In some cases the probiotic bacterium, lysate or composition is applied after exposure to the infective agent.

### Barrier effects

The probiotic bacteria and lysates of the present invention have barrier repair, reinforcement and/or regeneration activity. Skin is composed of two compartments, the deep compartment (the dermis) and the surface compartment (the epidermis). This constitutes a barrier against external attacks, in particular chemical, mechanical or infectious attacks, and, therefore, a number of defensive reactions against environmental factors (climate, ultraviolet rays, tobacco, etc) and/or xenobiotic, such as, for example, microorganisms, occur therein. This property is referred to as barrier function and is mainly provided by the most superficial layer of the epidermis, namely the horny layer, referred to as the stratum corneum. Detrimental changes in the barrier can be reflected by cutaneous discomfort, sensory phenomena and in particular unpleasant phenomena, or cutaneous dryness measured by imperceptible water loss. Compositions according to the invention are useful for preventing a reduction in the barrier and/or to repair or regenerate barrier function. Reduction of barrier function is associated with a number of disorders, such as those set out below. If the barrier is breached, for example by wounds or abrasions, the skin must repair itself. However, occasionally, repair does not occur quickly enough and this leaves the skin open to infection.

Disorders associated with disruption of the skin barrier include Psoriasis, Acne (including Hiradenitis Suppurativa), Burns, Diaper Rash, Netherton's Syndrome, Actinic Keratosis, Dermatomycoses, Dermatosis or Ectodermal Dysplasia, Atopic Dermatitis, Contact Dermatitis, Dermatitis, Seborrehic Dermatitis, Icthyosis Vulgaris or other disorders associated with damage or breakdown of the skin barrier

### Eczema/Dermatitis

Eczema (atopic dermatitis) is a particular type of inflammatory reaction of the skin in which there are typically vesicles (tiny blister-like raised areas) in the first stage followed by erythema (reddening), edema (swelling), papules (bumps), and crusting of the skin followed, finally, by lichenification (thickening) and scaling of the skin. Eczema characteristically causes itching and burning of the skin.

Eczema is very common and can first occur at any age. It is frequently chronic and difficult to treat, and it tends to disappear and recur. Itching can be extreme and severe.

There are a number of types of eczema. The prevalence of eczema is high with an estimated 33 million people in the USA alone affected.

### Ichthyosis

Ichthyosis vulgaris is a genetic skin disease that is characterised by the presence of excessive amounts of dry surface scales on the skin caused by abnormal epidermal differentiation or metabolism. It is usually most severe over the legs but may also involve the arms, hands, and trunk in some cases. It may also be associated with atopic dermatitis, keratosis pilaris (small bumps on the back of the arms), or other skin disorders. It usually disappears during adulthood, but may recur when elderly.

Other types of ichthyosis include X-linked ichthyosis, ichthyosis lamellaris, epidermolytic hyperkeratosis, harlequin type ichthyosis, Netherton's syndrome and Sjogren-Larsson syndrome, although ichthyosis vulgaris, accounts for 95% of all ichthyosis cases. Hereditary (congenital) ichthyosis vulgaris accounts for more than 95% of cases of ichthyosis vulgaris It first appears in early childhood. The gene responsible for ichthyosis vulargaris has been mapped to chromosome band 1q21. The product of this gene is a substance called filaggrin (FLG) which may act as the "keratin matrix protein" in cells of the stratum corneum. The inheritance pattern is autosomal dominant. Acquired ichthyosis vulgaris, typically develops in adulthood and results from an internal disease or the use of certain medications. Hereditary ichthyosis vulgaris is a common disease in United States, with a prevalence of approximately 1 case in 300 persons. Because symptoms improve with age, the true prevalence is probably higher. Acquired ichthyosis is extremely rare. Its prevalence in United States is unknown. In the United Kingdom, the incidence of ichthyosis vulgaris was reported to be 1 in 250. In China, ichthyosis vulgaris has a prevalence of 2.29%.

### Psoriasis

Psoriasis is a chronic, genetic, non-contagious skin disorder characterised by itchy or sore patches of thick, red skin with silvery scales. The scaly patches caused by psoriasis, called psoriatic plaques, are areas of inflammation and excessive skin production. The disorder is a chronic recurring condition which varies in severity from minor localised patches to complete body coverage. Psoriasis can also cause inflammation of the joints, which is known as psoriatic arthritis. There are a number of forms of the disease. According to the National Institutes of Health (NIH), as many as 7.5 million Americans (approximately 2.2 percent of the population) have psoriasis, with an estimated 125 million being affected globally. Between150,000 and 260,000 new cases of psoriasis occurring each year. Incidence of psoriasis tends to be affected by the climate and genetic heritage of the population. It is less common in the tropics and in dark-skinned persons, and most common in Caucasians. Total direct and indirect health care costs of psoriasis for patients are calculated at $11.25 billion annually, with work loss accounting for 40% of the cost burden¹⁶. Approximately 60% of psoriasis patients missed an average of 26 days of work a year due to their illness ¹⁷.

### Wound healing

As used herein wound healing refers to the process by which the skin repairs itself after injury. In normal skin, the epidermis (outermost layer, predominantly made up of keratinocytes) and dermis (inner or deeper layer) exists in a steady-state equilibrium, forming a protective barrier against the external environment. Once the protective barrier is broken, i.e. by wounding, the normal (physiologic) process of wound healing is immediately set in motion. As used herein the term "wound" refers to a type of injury in which the skin is torn, cut or punctured, or where the skin is damaged following a blunt force trauma or medical condition, resulting in a breach in the skin barrier. The probiotic bacteria or lysates thereof according to the invention may be useful in wound healing *in vivo,* including tissue repair, regeneration and/or replacement. For example, they may be used in healing scar tissue, acceleration of wound healing, or where tissue grafts such as skin grafts are used.

In some embodiments repair or regeneration of the skin barrier includes repair or regeneration of a mucous membrane. Mucous membranes include mucosa of the mouth (including mucosa of the cheek, the soft palate, the tongue, including the under surface of the tongue and the floor of the mouth), the nose, the throat (including mucosa of the pharynx, the larynx, the trachea and the esophagus), the bronchi, the lungs, the eye, the ear, the vagina, the penis, the urethra, the bladder, and the anus.

The patient to be treated may be any animal or human. The patient is preferably a non-human mammal, or a human patient. The patient may be male or female.

### Cosmetic Applications

In some aspects the invention relates to a cosmetic treatment comprising the administration of a probiotic bacterium or lysate according to the invention. "Cosmetic" as used herein is non-therapeutic. Such methods do not involve the treatment of the human or animal body by therapy. The cosmetic treatment may be used to improve the appearance and/or texture of the skin.

The invention also contemplates the use of probiotic bacteria or lysates thereof in a method of cosmetic treatment, and methods of cosmetic treatment using the probiotic bacteria or their lysates. For example, in a method of improving the hydration level, or appearance of the skin. As used herein the term "cosmetic method" does not refer to a method for treatment of the human or animal body by surgery or therapy, or diagnostic methods practised on the human or animal body according to Article 53(c)EPC.

The invention also provides a cosmetic composition comprising a probiotic bacterium. The composition may be used to improve the appearance of the skin. For example, the composition may enhance the hydration level of the skin. Increased hydration level is known to improve the appearance of the skin and lead to a healthier cosmetic appearance. Cosmetic compositions may be formulated similarly to pharmaceutical compositions, as described below.

The subject to be treated may be any animal or human. The subject may be a non-human mammal, but is more preferably human. The subject may be male or female. The subject does not require repair of their skin barrier. In some cases the subject does not require treatment for an infection, such as a bacterial infection. In some cases the subject does not require treatment at the site at which the cosmetic treatment is to be applied.

The cosmetic methods according to the invention preferably involve the administration of a "cosmetically effective amount". This pertains to the administration of compounds, ingredients, materials, compositions, dosage forms, etc. in an amount effective to induce a cosmetic benefit. This is within the scope of sound judgement of a relevant practitioner. It will be appreciated by one of skill in the art that appropriate dosages of the active compounds, and compositions comprising the active compounds, can vary from subject to subject.

Probiotic bacteria, lysates thereof, and compositions according to the invention have barrier maintenance and repair activity. As such, they are useful for preventing a reduction in and/or reinforcing barrier function. This may be useful for improving hydration of the skin and/or improving the appearance of the skin.

### Optionally disclaimed uses

The following represent independent optional and separable embodiments which in some cases may not form part of the present invention.

Optionally, some embodiments of the present invention do not include the combination of a probiotic bacterium or lysate thereof with a *Streptococcus* strain, e.g. an α-*Streptococcus* strain.

Optionally, some embodiments of the present invention do not include the combination of a *Lactobacillus rhamnosus* GG (or a lysate thereof) with *Lactobacillus rhamnosus* LC705 (or a lysate thereof).

Optionally, some embodiments of the present invention do not include cosmetic or dermatological treatment of dry and/or sensitive skin.

Optionally, in some embodiments methods of treatment involving repair or regeneration of the skin barrier are not for the treatment of an allergic disease/condition or inflammatory disease/condition. For example, such embodiments optionally do not include treatment of one or more of atopic dermatitis, seborrhoeic dermatitis, eczema, atopic eczema, ichthyosis, psoriasis, acne or allergic disease, or inflammatory conditions.

Optionally, some embodiments of the present invention do not include anti-inflammatory or anti-viral use of the probiotic bacterium, or lysate thereof.

Optionally, some embodiments of the present invention do not include the treatment of respiratory infections.

### Lysates

The probiotic bacteria according to the invention may be administered as live bacteria, an inactivated or killed bacterium, or as lysates thereof. As used herein "lysates" refers to a sample of the probiotic bacterium which has been subject to lysis. A lysate may contain one or more soluble metabolites of the probiotic bacterium useful in the methods described herein. Lysis may occur by chemical or physical disruption, for example by addition of an osmotic agent or enzyme to the bacteria, or by the application of physical pressure, for example through sonic disruption. Gueniche et al., for example, prepared a lysate by ultrasound (20).

The bacteria may be washed prior to lysis. The lysate may be filtered and/or sterilized after lysis to remove any whole bacteria remaining. The lysate may comprise a cell free supernatant. The lysate may be a mixture comprising the cellular contents of the probiotic bacterium. For example, one or more of fragments of cell wall or cell membrane, proteins, nucleic acids, carbohydrates, and organelles (disrupted or intact). The lysate may be suspended, for example in aqueous medium.

Lysed cells may allow the release of many substances, e.g. soluble metabolites, from inside the bacterial cell membrane that may contribute to bioactivity. Therefore in some cases use of a cell-lysate may be advantageous over the use of whole bacteria.

In some embodiments a cell free supernatant is the supernatant of a cell culture from which the cells have been removed. Cells may be removed by centrifugation, and the supernatant may be filtered. As such, the supernatant (as a form of lysate) may be used directly in the formulations of the present invention. A cell free supernatant may contain one or more soluble metabolites.

### Compositions

A composition may be administered alone or in combination with other treatments, either simultaneously or sequentially dependent upon the condition to be treated.

The probiotic bacterium or lysate thereof may be dissolved in, suspended in, or admixed with one or more other pharmaceutically acceptable ingredients. The probiotic bacterium or lysate thereof may be presented in a liposome or other microparticulate.

In some embodiments, probiotic bacterium may be provided as a suspension in a pharmaceutically acceptable excipient, diluent or carrier. In some embodiments probiotic bacterium may be provided as a lyophilisate.

In particularly preferred embodiments the probiotic bacteria or lysate thereof according to the invention is formulated for topical administration, particularly for use or application to, or on, the skin.

Formulations suitable for transdermal administration include gels, pastes, ointments, creams, lotions, and oils, as well as patches, adhesive plasters, bandages, dressings, depots, cements, glues, and reservoirs.

Ointments are typically prepared from the probiotic bacteria or lysate thereof and a paraffinic or a water-miscible ointment base.

Creams are typically prepared from the probiotic bacterium or lysate and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

Emulsions are typically prepared from the probiotic bacterium or lysate and an oily phase, which may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulphate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Other formulations include dental sprays, mouthwashes, toothpastes, lozenges, antibacterial washes, drinks (e.g. milk, yoghurt), food items (such as yoghurt, ice cream, candy bars), or powdered foods (such as powdered milk).

Formulations may suitably be provided as a patch, adhesive plaster, bandage, dressing, or the like which is impregnated with, or coated with, one or more probiotic bacteria or lysate thereof according to the invention and optionally one or more other pharmaceutically acceptable ingredients, including, for example, penetration, permeation, and absorption enhancers. The probiotic bacteria or lysates may also be provided in the form of coatings for medical devices such as implants, prosthetics, surgical instruments, gloves, catheters, valves, pacemakers and the like.

A formulation may contain a single (unit) dose of probiotic bacteria, or lysate thereof. Suitable doses of probiotic bacteria (intact or lysed) may be in the range 10⁴ to 10¹² cfu, e.g. one of 10⁴ to 10¹⁰, 10⁴ to 108, 10⁶ to 10¹², 106 to 10¹⁰, or 10⁶ to 10⁸ cfu. In some embodiments doses may be administered once or twice daily.

In some embodiments a formulation for use according to the present invention may comprise at least about 0.01 %, about 0.05%, about 0.1 %, about 0.2%, about 0.3%, about 0.4%, about 0.5%, about 0.6%, about 0.7%, about 0.8%, about 0.9%, about 1.0%, about 1.5%, about 2.0%, about 3.0%, about 4.0%, about 5.0%, about 6.0%, about 7.0%, about 8.0%, about 9.0%, about 1 0.0%, about 11.0%, about 12.0%, about 13.0%, about 14.0%, about 15.0%, about 16.0%, about 17.0%, about 18.0%, about 19.0%, about 20.0%, about 25.0%, about 30.0%, about 35.0%, about 40.0%, about 45.0%, about 50.0% by weight of probiotic bacteria or lysate thereof.

In some embodiments the formulation may comprise, one of at least about 0.01% to about 30%, about 0.01% to about 20%, about 0.01% to about 5%, about 0.1% to about 30%, about 0.1% to about 20%, about 0.1% to about 15%, about 0.1% to about 10%, about 0.1% to about 5%, about 0.2% to about 5%, about 0.3% to about 5%, about 0.4% to about 5%, about 0.5% to about 5%, about 1% to 10 about 5%, by weight of probiotic bacteria or lysate thereof.

### Antibacterial compositions

The invention also provides antibacterial compositions in the form of cleaning products, washes, surface coatings or other compositions which are not for medical treatment of the human or animal body.

Such agents may be useful for removing, killing, or preventing the accumulation of bacteria on a surface, or inhibiting the action or growth of the bacteria.

Anti-bacterial compositions according to the invention may be useful for treating biomaterials, implants and prosthesis (including stents, valves, eyes, hearing aids, gastric bands, dentures, artificial joint replacements etc), surgical instruments or other medical devices prior to administration to, or treatment of, or use with, a patient or subject. The antibacterial compositions may be useful for treating surfaces prone to colonisation or exposure to bacterial, such as handrails, food preparation surfaces, kitchen surfaces or equipment, tables, sinks, toilets or other bathroom hardware,

Antibacterial compositions may comprise agents in addition to the lysate, such as cleaning agents, stabilisers, anionic surfactants, perfumes, chelating agents, acids, alkalis, buffers or detergents. Such agents may facilitate or enhance the antibacterial properties of the agent, such as killing or inhibiting bacteria, or preventing the recolonisation of the cleaned surface.

### Methods for preparing compositions

The invention also relates to methods of preparing compositions. The method may comprise subjecting a population of probiotic bacteria to lysis and formulating the resulting lysate into a composition. The method may comprise the step of treating the lysate so as to remove or inactivate intact bacteria in the lysate, for example, by partitioning intact bacteria from the lysate. The lysate may be subject to purification of filtration steps to remove other components, such as bacterial growth media or contaminants.

In alternative compositions intact bacteria are formulated as a composition. The bacteria may be inactivated prior to, or after, formulation into a composition. The bacteria may be subject to a purification or filtration step so as to remove other components, such as bacterial growth media or contaminants.

The compositions prepared by such a method may be suitable for use in treatment, for example in treating bacterial infections, or for repairing the skin barrier. Such methods may involve the addition of one or more pharmaceutically or cosmetically acceptable excipients.

Other compositions may be not suitable for use in treatment, for example antibacterial compositions such as washes. Methods for the preparation of antibacterial compositions may involve the addition of one or more agents to the lysate or bacteria.

### Example 1: Materials and Methods

### Growth of bacteria

Probiotic bacteria were grown routinely to stationary phase in Wilkins-Chalgren Broth (WCB) (Oxoid) at 37°C in a Mark 3 Anaerobic Work Station (Don Whitley Scientific, Shipley, UK). S. *aureus* was grown aerobically with at 37°C in Nutrient Broth (Oxoid). Culture densities were adjusted spectrophotometrically with medium to contain the number of bacteria required. For experiments utilising keratinocytes, bacteria were washed twice in 0.85% NaCl solution, centrifuged and reconstituted in keratinocyte medium. Selective agar was used in some experiments. This was Mannitol Salt Agar-MSA (Oxoid) or Man-Rogosa Sharpe agar (MRS) for S. *aureus* or Lactobacilli respectively. For experiments utilising heat killed bacteria, *L. reuteri* was centrifuged and resuspended in 0.4% glucose and heat inactivated by placing in a water bath at 85°C for 45 minutes. Samples were gram stained to ensure lysis of bacterial cells had not occurred, and plated onto growth media to ensure they had been killed. For experiments using probiotic lysates, 10ml of 10⁸ cfu/ml *L. reuteri* was centrifuged, washed, concentrated in 1ml keratinocyte media and lysed using a bead beater (FastPrep™ FP120, Thermo Electron Corporation). Samples were filter sterilised to remove any whole bacteria remaining. Approximately 100µl of this lysate was used to treat keratinocytes.

### Mammalian Cell Culture

Normal human epidermal keratinocytes (NHEK) (Promocell, Heidelberg, Germany) were maintained in keratinocyte basal medium (Promocell, Heidelberg , Germany) containing a supplement mix (bovine pituitary extract 0.004mg/ml, epidermal growth factor (recombinant human) 0.125ng/ml, insulin (recombinant human) 5µg/ml, hydrocortisone 0.33µg/ml, epinephrine 0.39µg/ml and transferrin, holo (human) 10µg/ml) and 0.06mM CaCl₂ (Promocell Heidelberg, Germany). Medium was substituted twice weekly and cells were cultured at 37°C in a humid atmosphere of 5% CO₂. Cells were detached once grown to 80% confluency and re-seeded at approximately 5 x 10³ cells/cm². For experiments using differentiated cells, calcium chloride in the medium was increased to 1.8mM and the cells were grown in this for 24 hours prior to experimentation.

### Bacterial competition assay

Aliquots (100µl) of overnight cultures of *L. reuteri* and S. *aureus* were inoculated into fresh 10ml broths (both as axenic cultures and as co-cultures). The pH and optical density of cultures was measured at 0 and 48 hours. At regular intervals (3, 6, 24, 30, and 48 hours), bacteria were counted by serial dilution plate counts using selective and non-selective agars. At 48 hours, BacLite ™ Live/Dead stains were performed according to the manufacturer's instructions (Invitrogen).

### Production of inhibitory substances by L. reuteri

Inhibition of the growth of S. *aureus* by *L. reuteri* was determined using well diffusion assays, as described by Holder and Boyce (23). For inhibition studies, *L. reuteri* was grown in either Man-Rogosa-Sharpe (MRS) media (Oxoid) or Wilkins-Chalgren Broth (WCB) (Oxoid) anaerobically at 37°C to stationary phase. Whole cell (WC) cultures of *L*. *reuteri* grown in WCB (1g/l glucose) or MRS (20g/l glucose) were used. In other experiments cells were sedimented in a microfuge at 15,000 x g for 5 minutes and the cell-free supernatant (CFS) extracted for use. The plates were then incubated at 37°C for 48h and zones of inhibition calculated. To measure the production of organic acids by *L. reuteri,* the pH of WC and CFS was measured. In a separate experiment to determine whether *L. reuteri* produced any other inhibitory substances, cell free supernatants were neutralised using 1M NaOH prior to use in the well diffusion assay. To determine if *L*. *reuteri* produced organic acids in cell culture, the pH of infected cell cultures was measured.

### Measurement of keratinocyte viability

NHEK cells were grown in 24 well tissue culture plates to confluency. Cell culture medium was replaced with medium containing the indicated concentration of bacteria. For pre-exposure and post-exposure experiments, NHEK were treated with *L. reuteri* for periods of between 1 and 4 hours prior to the addition/after the addition of *S. aureus.* In all experiments, cells were incubated at 37°C in a humidified atmosphere of 5% CO₂ for 24 hours. The medium was then removed and cells were washed with sterile PBS. Cells were detached using trypsin (0.4%) / EDTA (0.3%) (Promocell) and cell viability determined by trypan blue exclusion assay (45).

### Measurement of S. aureus viability

To determine if *L. reuteri* or NHEK were able to inhibit the growth of *S. aureus* in cell culture, cells were grown to confluency in a 24 well plate. These were infected with either S. *aureus* or *S. aureus* and *L. reuteri* together. In parallel, wells without any cells also had these combinations added to determine the effect of the keratinocytes themselves on staphylococcal viability. After 24 hours exposure, the medium was removed and centrifuged to pellet extracellular bacteria. The cells were then trypsinised and 500µl of 0.25% Triton-X-100 (Sigma-Aldrich) in PBS was added for approximately 30 minutes to lyse the cells. The well contents were then combined with the cell pellet and serial dilution plate counts using Mannitol Salt Agar (MSA) performed to determine the total number of viable staphylococci.

### Adhesion Assays for L. reuteri, L. salivarius and S. aureus

Confluent differentiating NHEK were exposed to either the probiotic or *S. aureus* for 1 hour. After incubation, cells were washed three times in PBS to remove non adherent bacteria. The cells were trypsinised and serial dilution plate counts performed to assess the number of adherent bacteria. Selective agar was used for growth of staphylococci. In separate experiments, cells were exposed to *L. reuteri* 1 hour before the addition of *S. aureus* (exclusion), at the same time (competition) or 30 minutes after *S. aureus* infection had begun (displacement) to determine whether *L. reuteri* could inhibit *S. aureus* adhesion to keratinocytes. To determine whether *L. reuteri* could inhibit invasion of keratinocytes by staphylococci, cells were either exposed to *S. aureus* or *S. aureus* and *L. reuteri* for 1 hour. Cells were washed three times in sterile PBS to remove non-adherent bacteria, and the growth medium replaced with medium containing 100µg/ml Gentamicin (Sigma-Aldrich) for 2 hours. Cells were then washed three times in sterile PBS and trypsinised and lysed in 0.25% Triton-X-100 for 30 minutes to release internalised bacteria. Bacteria in lysates were counted using serial dilutions. In experiments utilising conditioned media (CM), NHEK were exposed to *L. reuteri* and incubated for 1 hour at 37°C in 5% CO₂. The CM from the exposed cells was removed and centrifuged at 15000 x g for 3 minutes to pellet the bacterial cells and filtered through a 0.22µm pore size filter (Millipore, USA). In experiments exploring the role of the α5β1 integrin in *S. aureus* adhesion, NHEK were pre-exposed to 60µg/ml of an anti- α5β1 integrin antibody (Millipore, USA) for 1 hour prior to infection of cells. An alternative method for assessing the number of adherent bacteria was utilised for dead preparations of *L.reuteri.* Cells were grown on LabTek chamber slides (Thermo-Scientific). Adhesion assays were performed as usual, using adjusted amounts of bacteria. After the cells were exposed to bacteria, cells were washed twice in PBS and fixed in methanol for 20 minutes before performing a gram stain. The number of adherent bacteria per 100 cells was then assessed using a Keyence All in one Type Fluorescence microscope.

### Statistical analyses

All experiments were performed a minimum of three times, with three replicates each time. For experiments comparing two treatments, a students T-test was used. For experiments comparing two or more treatments, a one way ANOVA with blocking by experiment and post hoc Tukey test was utilised. Dose response and competition assays were analyzed using linear regression and 2-way ANOVA respectively. Results were considered significant if P<0.05.

### Example 2

### S. aureus induced cell death in primary human keratinocytes

We initially characterised the effects of *S. aureus* on keratinocyte viability by performing trypan blue exclusion assays on NHEK incubated with *S. aureus* at different doses. At a concentration of 10⁵ cfu/ml *S. aureus,* only 49.4% of keratinocytes were viable after 24 hours infection (Fig. 1). This was reduced in a dose dependent manner such that only 3.3% of keratinocytes were viable at concentrations of 10⁸ cfu/ml *S. aureus* (Fig. 1). Approximately 10⁶ organisms is a physiologically relevant concentration that might be found e.g. in a skin wound infection (7). At this concentration, 30.5% of keratinocytes were viable after 24 hours infection. This concentration was used in adhesion assays.

### L. reuteri protects keratinocytes from S. aureus induced cell death

The ability of three strains of lactobacilli (*L. reuteri* ATCC 55730, *L. rhamnosus* AC413 and *L. salivarius* UCC118) to protect keratinocytes from the effects of S. *aureus* was investigated (Fig. 2a). Infection of NHEK with S. *aureus* reduced viability to 8.8 %. In contrast to S. *aureus,* incubation of NHEK with lactobacilli did not result in significant reduction in cell viability (Fig. 2a). Co-infection with S. *aureus* and either *L. rhamnosus* or *L. reuteri* resulted in increased keratinocyte viability at 24 hours of 42.7 % and 53.1% (P=0.0012 and P<0.0001) respectively. This level of protection was observed whether the cells were differentiated for 24 hours in calcium, or undifferentiated (Results not shown). However, co-infection of keratinocytes with S. *aureus* and *L. salivarius* did not result in a significant elevation in viability compared to NHEK infected with S. *aureus* (P=0.052, Fig. 2a). Since *L. reuteri* was more protective than the other two strains, we further investigated the effects of *L. reuteri* by asking whether the protective effect of *L. reuteri* was dose dependent. However, there was no further significant increase in keratinocyte viability using *L. reuteri* at 10⁵, 10⁶, 10⁷ or 10⁸ cfu/ml (P>0.05) (Fig. 2c). We also investigated whether the protective effects of the probiotic were dependent on live bacteria. Although heat killed *L. reuteri* did not protect NHEK from *S. aureus,* an *L. reuteri* lysate afforded a significant protective effect (P=0.01) (Fig. 2d and e, respectively). Further experiments utilised 10⁸ cfu/ml of live *L. reuteri* (MOI of 1000).

### The protective effects of L. reuteri are time dependent

We next investigated whether the timing of *L*. *reuteri* application relative to *S. aureus* infection was critical to the protective effect of the probiotic. We assessed the viability of keratinocytes pre- or post-exposed to *L. reuteri* for 1, 2 or 4 hours prior to infection with *S. aureus* for 24 hours. Pre-exposed keratinocytes had similar viabilities to co-infected cells (P>0.05) (Fig. 3a). However, keratinocytes exposed to *L. reuteri* 1, 2 or 4 hours after *S. aureus* infection had commenced had similar viabilities to cells exposed to S. *aureus* (P>0.05) (Fig. 3b).

### L. reuteri does not inhibit growth of S. aureus

We considered that the mechanism(s) underlying the protective effects of *S. aureus* might be due to direct effects of the probiotic on the pathogen. However, competition assays showed no significant reduction in either *L. reuteri* or *S. aureus* viability during co-culture for 48 hours (Fig. 4a) compared to axenic cultures (P>0.05). Additionally, in keratinocyte culture assays, the total number of viable staphylococci was also unaffected by the presence of the probiotic (Figure 4b; Table 1). A live-dead count of the co-culture at 48 hours also demonstrated no significant reduction in bacterial cell viability (data not shown). Furthermore, although we established that *L. reuteri* could produce organic acid that inhibited pathogen growth on agar plates (data not shown), when we measured the pH of keratinocyte media infected with *S. aureus, L. reuteri* and both simultaneously for 24 hours, there was no significant difference in the pH between treatments.

### L. reuteri inhibits adhesion and invasion of staphylococci to keratinocytes

One of the mechanisms by which probiotics are known to protect gut epithelia is by inhibition of pathogenic adhesion to epithelial cells. Therefore, we explored whether *L. reuteri* could inhibit adhesion of *S. aureus* to NHEK.

The graph in Fig. 5a demonstrates that both *L. reuteri* and *S. aureus* adhered to NHEK. Next we established whether *L. reuteri* could exclude or compete with *S. aureus* for binding sites by adding the probiotic either before, or at the same time as the pathogen. The graphs in Fig. 5b and 5c demonstrate a reduction in adhesion of *S. aureus* to NHEK when *L. reuteri* was added before, or at the same time as addition of the pathogen (P=0.026 and P=0.0078 respectively). However, if *L. reuteri* was added after *S. aureus,* the probiotic could not prevent the pathogen from adhering to (P>0.05, Fig. 5d). Furthermore, gentamicin protection assays demonstrated that the probiotic inhibited *S. aureus* invasion of NHEK (P=0.009, Fig. 5e). Our results showed that heat killed *L. reuteri* did not significantly inhibit staphylococcal adhesion (P>0.05, Fig. 6a). However, probiotic lysates were able to inhibit staphylococcal adhesion (P= 0.032), though not to the same extent as live *L. reuteri* (P=0.0002). NHEK had approximately 5.6 ± 0.2 log cfu staphylococci adhered when exposed to live *L. reuteri* compared to 6.0 ± 0.2 log cfu when exposed to lysates (Fig. 6b).

### L. salivarius UCC118 does not prevent S. aureus from adhering to keratinocytes

We considered that the protective effects of *L. reuteri* might be connected to its ability to inhibit pathogen adhesion to NHEK. To further explore this, we asked whether inhibition of *S. aureus* binding to NHEK resulted in a decrease in its toxicity. S. *aureus* is known to adhere to cells by means of a fibronectin binding protein which interacts with extracellular fibronectin (26, 36). Fibronectin itself binds to the keratinocyte receptor, the α5β1 integrin. As shown in previous studies (27) pre-treatment of cells with a blocking antibody to α5β1 integrin resulted in decreased *S. aureus* adhesion. Therefore, we treated NHEK with the α5β1 blocking antibody and investigated its ability to inhibit adherence and toxicity to NHEK (Fig. 7a and b). The blocking antibody significantly inhibited *S. aureus* adhesion to NHEK (P=0.007), while it also appeared to inhibit the ability of *S. aureus* to induce cell death in NHEK (P=0.03). Next, we investigated the ability of *L. salivarius,* which did not protect NHEK, (Fig. 2a) to inhibit *S. aureus* adhesion. Keratinocytes exposed to *L*. *salivarius* and S. *aureus* were found to have similar numbers of adherent staphylococci (4.5 ± 0.2 log cfu) as keratinocytes exposed to *S. aureus* only (4.9 ± 0.2 log cfu) (P>0.05, Fig. 8a). Furthermore, *L. salivarius* did not adhere to NHEK as efficiently (5.6 ±0.1 log cfu) as *L. reuteri* (6.7 ± 0.1 log cfu) (P=0.005, Fig. 8b).

### DISCUSSION

In this study, we have made a preliminary step towards characterizing the utility of probiotics for topical use. We have investigated the ability of three strains of lactobacilli with known probiotic potential in the gut and mouth (9, 38, 44) to protect keratinocytes from the effects of the common skin pathogen, *S. aureus.*

The application of *S. aureus* at physiologically relevant concentrations killed 69.5% of NHEK within 24h. By contrast, none of the strains of lactobacilli significantly affected the viability of NHEK under the same conditions, suggesting that probiotics were well tolerated by keratinocytes. When NHEK were exposed to both pathogen and *L. reuteri,* the probiotic protected the cells from the effects of the pathogen, as indicated by the significantly higher percentage viability of *S. aureus* infected NHEK in the presence vs. absence of *L. reuteri* (Fig. 2a and b). The protective effect was also observed with lysates of *L. reuteri,* but not heat-killed *L. reuteri* cells (Fig. 2d and e). Furthermore, the protective effects of the probiotic were variable between different species because the protection afforded by *L. reuteri* was greater than that provided by *L. rhamnosus.* By contrast, *L*. *salivarius* did not provide keratinocytes with any significant protection from *S. aureus* (Fig. 2a).

While, to our knowledge, this is the first evidence showing the ability of a probiotic to protect keratinocytes from the pathogenic effects of *S. aureus in vitro, L. fermentum* has been demonstrated to inhibit S. *aureus* infection of surgical implants and abscess formation in rats (18). Probiotics have previously been shown to exert protective effects against pathogen-induced cell death in other epithelia. For example, *L. rhamnosus, L. reuteri* and *L. oris* were shown to inhibit *S. pyogenes* induced cell death of pharyngeal epithelial cells (34). Probiotic cell free supernatants have also been shown to be protective. *L. delbruekii* supernatants protect enterocytes from *C. difficile* pathogenicity (5).

The protective effect of *L. reuteri* against *S.* aureus-induced cell death of keratinocytes could result from several different mechanisms. Lactobacilli are known to produce organic acids and bacteriocins that can have direct antimicrobial effects on pathogens. Although we could demonstrate in diffusion assays that *L. reuteri* could produce acid, this does not appear to be responsible for the protective effects observed because the pH of the keratinocyte culture medium did not change in the presence of lactobacilli. Similarly, we did not detect any other antimicrobial activity since in a competition assay, the growth rate and productivity of *S. aureus* in the presence of *L. reuteri* was identical to that of axenic cultures (Fig. 4).

At least part of the mechanism by which *L. reuteri* protects keratinocytes appears to be linked to the ability of *L. reuteri* to prevent adhesion of *S. aureus.* Several lines of evidence point to this: Firstly, S. *aureus* was less toxic to the cells when its adhesion was inhibited using an integrin blocking antibody (Fig. 8a and b). Secondly, lysates of *L. reuteri* that inhibit adhesion were also protective (Fig.2e and 6b). On the other hand, heat killed *L. reuteri* did not protect keratinocytes (Fig. 2d). Thirdly, strains of lactobacilli that did not inhibit *S. aureus* adhesion *(L. salivarius*) (Fig. 8a) did not protect keratinocytes (Fig. 2a).

The timing of *L. reuteri* addition to the cells appears to be critical to its protective effects. When *L. reuteri* was added before, or at the same time as *S. aureus,* NHEK were protected from the effects of the pathogen (Fig. 3a and 2a respectively). However, if the probiotic was added after the pathogen, the levels of keratinocyte cell death were similar to that seen in cultures not treated with probiotic (Fig. 3b). Taken together, our data point to a mechanism of protection by *L. reuteri,* involving competitive exclusion of *S. aureus* from keratinocyte binding sites.

How *S. aureus* induces cell death in keratinocytes is a complex process. In general, the first step in *S. aureus* pathogenesis is considered to be adherence of *S. aureus* to keratinocytes prior to invasion (27). *S. aureus* utilises a number of adhesins to do this, collectively called the Microbial Surface Components Recognising Adhesive Matrix Molecules (MSCRAMMS) (11). Staphylococcal adhesins known to be involved in adhesion to keratinocytes include Fibronectin binding proteins (FnBPs) (27, 36), protein A, clumping factors and coagulase (35). Teichoic acid mediates binding of *S. aureus* to nasal epithelial cells, though whether this is true for keratinocytes is not known (1). However, integrin blocking did not wholly inhibit binding of *S. aureus* to NHEK, suggesting that more than one adhesin is used (Fig. 8a).

Our study and others (27) have shown that blocking fibronectin receptors on the keratinocytes inhibits adhesion of S. *aureus* (Fig. 8a). Interestingly, in separate experiments (data not shown) we could not show involvement of this integrin in binding of *L. reuteri* to NHEK. Indeed, neither protease nor glycosidase treatment of keratinocytes was able to prevent *L. reuteri* from binding to cells (data not shown). However, whatever the mechanism is by which the probiotic binds to cells, it involves a heat labile molecule, as heat killed *L. reuteri* do not adhere to keratinocytes as efficiently (data not shown), nor protect them from *S. aureus* (Fig. 2d).

To date, there are no published reports on the ability of probiotics to adhere to epidermal keratinocytes. Adhesion of probiotics to epithelia elsewhere has been suggested to occur via collagen binding proteins (21), mucus binding proteins (37), lipoteichoic acids (19) and s- layer proteins (10, 14). The relevant adhesins may be proteinacious, carbohydrate or a mixture of the two, and more than one adhesin may be in use. *L. reuteri* was able to inhibit staphylococcal adhesion to keratinocytes when cells were pre-exposed to the probiotic (exclusion of binding sites) (Fig. 5b) and co-infected with *S. aureus* (competition for binding sites) (Fig. 5c) but not when applied after staphylococcal infection had begun (displacement from binding sites) (Fig. 5d). Others have shown that competitive inhibition/exclusion is often strain dependent, while displacement is generally not as effective or may require longer to occur (32, 49). Our results suggest that *L. reuteri* does not displace existing *S. aureus* from keratinocytes and support the finding that *L. reuteri* cannot protect keratinocytes from *S. aureus* cytotoxicity if added after infection has commenced. Some evidence has emerged to suggest that probiotics may have a synergistic effect on each others adhesion, suggesting an investigation into the effect of combinations of probiotics may be of interest in the future (39).

It seems likely that *L. reuteri* employs multiply mechanisms to attach to host epithelia and may use several strategies to prevent *S. aureus* from adhering. It is possible that *L. reuteri* may influence the expression of adhesive structures on the surfaces of keratinocytes, an area which has yet to be explored. Previous work has shown that probiotic *L. rhamnosus* GG and *L. plantarum* 299v can induce upregulation of genes involved in mucin expression in the intestine, which in turn inhibits adhesion of enteropathogenic *E. coli* (33). In a study in 1999, application of the lactic acid bacterium S. *thermophilus* to keratinocytes *in vitro* and skin *in vivo* resulted in increased ceramide production(15). Another study in 2006 used a probiotic fermented whey product on NHEK and found increased mRNA expression of the differentiation markers keratin-10 and involucrin (2) suggesting that "postbiotics" or metabolites of probiotics may also be of use for skin maladies. Another example of the positive effects of metabolites of probiotics was demonstrated in a recent paper where a probiotic patch for skin was developed. Lyophilised *L. fermentum* suspended in alginate with glucose and nitrite salts was able to produce nitric oxide gas, a substance able to inhibit the growth of pathogenic bacteria (24).

We have provided evidence that a probiotic has a protective effect against the pathogenic effects of *S. aureus* on keratinocyte viability *in vitro.* These results suggest that a topical probiotic for skin could exclude pathogenic organisms and therefore prevent infections, and agrees with the work of others. The topical administration of *L. plantarum* to burns in mice has been shown to inhibit *P. aeruginosa* colonisation of burns, promote healing and modify the inflammatory response (46). This organism has also been compared to and found to be as effective as traditional silver suphadiazine treatments of burn wounds for limiting the bacterial load of wounds and healing *in vivo* (41). In addition, a probiotic may also be of use in the treatment of healthy skin for increased moisturisation and barrier function. In 2010 a Bifidobacterium lysate was applied topically to the skin of volunteers and found to increase barrier function of skin through decreased trans-epidermal water loss measurements (20). While further investigations are required to determine the full effect probiotics have on the skin, our findings show that *L. reuteri* can protect keratinocytes from the pathogenic effects of *S. aureus* through the exclusion and competition for binding sites. We suggest that the prophylactic use of probiotics or their lysates in barrier creams and soaps may aid in the fight against staphylococcal skin colonisation and subsequent infections.

### Example 3

As set out above, we demonstrated that *Lactobacillus reuteri* can partially protect primary human keratinocytes from the effects of the skin pathogen, *Staphylococcus aureus.* In this study, to further our understanding of the potential of probiotics, we have evaluated the role of a second probiotic, *L. rhamnosus GG* as well as combinations of lactobacilli, and bacterial lysates in the inhibition of S. *aureus* infection of keratinocytes. *L.rhamnosus GG* was isolated from human faeces (see US4839281 and US5032399), and is deposited under accession number ATCC 53103.

### L. reuteri protects keratinocytes from S. aureus induced cell death

Normal human primary keratinocytes (KCs) were exposed to S. *aureus* (10⁶/ml) in the presence or absence of *L. rhamnosus GG, L reuteri* either alone or in combination (all at 10⁸/ml). The viability of the KCs was measured after 24 hours using a trypan blue exclusion assay.

When KCs were treated with *S. aureus* alone, only 39% of the KCs remained viable at 24 hours post infection (p<0.001). However, in the presence of either *L. reuteri* or *L*. *rhamnosus GG,* the viability of pathogen infected KCs increased to 60% and 59% respectively (p=0.008 and 0.009 respectively). Furthermore, when we used both strains in combination on *S. aureus* infected cells, the viability of the KCs increased to 75% (p=0.0025).

Interestingly, a lysate of *L rhamnosus GG* also provided significant protection to KCs with 70% of cells being viable following a 24 hour incubation with *S. aureus* in the presence of the probiotic lysate.

### L. rhamnosus inhibits growth of S. aureus

*S. aureus* was grown in the presence of *L. rhamnosus* GG or *L. rhamnosus* GG lysate. As shown in Figure 12 growth of *S. aureus* was slower in the presence of either live *L*. *rhamnosus* or lysate than *S. aureus* alone.

This inhibitory effect was not observed in *L. rhamnosus* AC413. As shown in Figure 11 in a well diffusion assay *S. aureus* growth was not inhibited as no zone of inhibition was visible around the well after 24 hours.

### L. rhamnosus kills S. aureus

To further elucidate the effect of *L. rhamnosus* GG on *S. aureus* we investigated the effect of *L. rhamnosus* or its lysate on *S. aureus* viability. As shown in Figure 13, in the presence of live *L. rhamnosus* GG a greater number of dead *S. aureus* cells were measured as compared to S. *aureus* grown in the absence of *L. rhamnosus* GG (LGG). The number of dead cells was dramatically increased when *S. aureus* was cultured in the presence of LGG lysate.

Thus, we have identified a strain of probiotic bacterium with skin cell protecting and regenerative properties. Significantly, lysates of this strain improve skin keratinocytes ability to resist harmful toxicity induced by *S. aureus* (SA) (Figure 9a). When used in combination with a second strain lysate, the combination is even more effective at protecting against *S. aureus* than when either strain is used alone (Figure 9b).

### L. rhamnosus GG and L. reuteri speed up recovery of a scratched keratinocyte monolayer and increase keratinocyte migration

Agents that increase and repair the barrier function of keratinocytes, have great potential not only as therapeutic agents but also as cosmetic skin improving agents. Augmentation of the barrier function increases the skins hydration level and is known to improve the appearance of the skin and lead to a healthier cosmetic appearance.

We have also demonstrated that when keratinocyte monolayers are scratched to remove cells (i.e. a model wound), the presence of our propriety probiotic lysates significantly and surprisingly speeds up the recovery of the monolayer and re-establishment of the barrier (Figure 10). As shown in Figure 14, *L. rhamnosus* GG or *L. reuteri* lysates increased the rate of re-epithelialisation of keratinocyte monolayers. This effect is not observed when lysates of *L. plantarum, L. fermentum, S. epidermidis,* or *Bifidobacterium longum* extracts are applied to a wounded keratinocyte monolayer.

LGG and *L. reuteri* lysates were also able to increase the rate of keratinocyte migration. As shown in Figure 15 only LGG and *L. reuteri* extracts were able to increase keratinocyte migration rate as compared to the control. Keratinocyte migration is an important mechanism by which monolayers re-epithelialise.

These data demonstrate that different strains of probiotic *Lactobacilli* either alone or in combination can protect human keratinocytes from the effects of the skin pathogen, S. *aureus.* Our data suggest that probiotics may potentially have applications, for example, in topical formulations to protect the skin from adventitious pathogens.

### Example 4

### Materials and Methods

### Preparation of probiotic lysates.

All probiotic strains (*B*. *longum reuterATCC-51870, L. plantarum,* ATCC-10241, *L. reuteri,* ATCC-55730, *L. rhamnosus* Goldwin and Gorbach, ATCC-53103, *L. fermentum,* ATCC-14932) were purchased from LGC ltd (UK) and were routinely grown to stationary phase in Wilkins-Chalgren Broth or on Wilkins-Chalgran agar at 37°c in a Mark 3 anaerobic work station (Don Whitley scientific, UK). Cultures were adjusted spectrophotometrically to contain 10⁸cfu/ml. 10 ml of these cultures was centrifuged, washed and then concentrated in 1 ml of keratinocyte basal medium (Promocell,Heidelberg, Germany) . The sample was then lysed using a bead beater (FastPrep FP120, Thermo Electron Corporation, UK) and then filter sterilised to remove any remaining whole bacteria. 100 µl of this lysate was used to treat keratinocyte cultures.

### Primary keratinocyte cell culture and measurement of TEER.

Normal human epidermal keratinocytes (NHEK) were obtained and cultured as described in Prince et al (50). For experiments measuring TJ function, cells were plated on 12- well, permeable polycarbonate Thincert™ cell culture inserts with 0.4um pore size (Greiner Bio-one Ltd, UK). NHEK were grown in keratinocyte basal medium (Promocell, Heidelberg, Germany) until confluent. At this point, the medium was replaced with CNT-02-3DP5 high calcium medium (CELLnTEC Advanced cell systems, Switzerland) which induces TJ formation. TJ function was measured using an epithelial voltmeter fitted with chopstick electrodes (World Precision Instruments Ltd, UK). All experiments were repeated at least three times with triplicate wells within individual experiments. In some experiments, lysates of probiotic bacteria were added to the apical side of the inserts and the TEER was measured at the times indicated post treatment.

### Measurement of NHEK viability using MTT assay.

3-{4,5-dimethylthiazol-2-yl}diphenyltetrazolium bromide (MTT, purchased from the Sigma-Aldrich Ltd., Poole, UK) was prepared as a stock solution of 5 mg/ml in phosphate buffered saline. NHEK were grown to confluence in 96 well plates and then treated with bacterial lysates for 24 hours. Medium was then replaced with fresh medium containing 10% MTT. The plates were incubated for 4 hours at 37°C and the medium was replaced with dimethyl sulphoxide. The absorbance of each well was then read at 570 nm in a plate reader.

### Extraction of protein from NHEK and immunoblotting.

Protein was extracted from NHEK cells according to the method described in Mclaughlin et al (51). Briefly, cells from a single Thincert™ were scraped into 100 µl extraction buffer (NaCl (120 mM), HEPES, pH 7.5 (25 mM), Triton X-100 (1%), EDTA (2 mM), NaF (25 mM), NaVO4 (1 mM), SDS (0.2%), Aprotinin (10 µg/ml), Leupeptin, (10 µg/ml) and pepstatin A (10 µg/ml) and then incubated on ice for 30 minutes. Following centrifugation in a microfuge, the supernatant was recovered to a fresh tube and used for analysis of TJ protein expression. SDS-PAGE was performed according to the method of Laemmli et al (52) and proteins were electrophoretically transferred onto PVDF membranes. These were subsequently washed, blocked in 5% (w/v) skimmed milk and incubated with the primary antibodies overnight. These were rabbit anti-claudin 1, 4, mouse anti occludin, mouse anti-ZO-1 or mouse anti-β-actin (all purchased from InVitrogen). The membranes were subsequently washed and incubated with horse radish peroxidise conjugated secondary antibodies. The immunoblots were developed using enhanced chemiluminescence (Amersham, Bucks, UK) and densitometry performed as described in Mclaughlin et al, 2004 (51).

### Inhibition of TLR-2.

For inhibition of TLR2, keratinocytes were pre-treated with recombinant human anti-TLR2 antibody at 10 µg/ml (Abcam, UK) for 1 hour before stimulation with probiotic lysates.

### Statistical analysis.

All experiments were performed a minimum of three times and analysed using SPSS software version 20. For experiments comparing two treatments, an Independent Samples T-test was used to statistically analyse all data. For experiments comparing two or more treatments, a one-way ANOVA test was used to statistically analyse all data. Results were considered statistically significant at p<0.05. Data are expressed as means ± standard errors of the means (SEM) for at least n=3 for each experiment.

### Results

### Probiotic lysates are well tolerated by NHEK.

To begin with, we investigated whether any of the probiotic lysates under investigated affected the viability of NHEK. To this end, we performed an MTT assay on NHEK that had been incubated with bacterial lysates for 24 hours. The data in Figure 16 illustrate that, with the exception of lysates of *L. fermentum,* none of the lysates of the strains tested significantly affected the viability of NHEK. The *L. fermentum* lysate induced a 32% (±1.3%, *p*=<0.01, *n*=3) reduction in NHEK viability following 24 hours incubation.

### Probiotic bacteria augment TJ function in NHEK.

Normal human epidermal keratinocytes develop TJs when they are transferred from medium containing low calcium concentrations (∼0.2mM) into medium containing high calcium (∼1.8mM). This 'calcium switch' induces assembly of TJs which can be detected as a rise in the TEER of the cells with time. TEER reaches a peak 48 hours post calcium switch and then drops slightly to reach a steady state at 72 hours (Figure 17a). When 100 µl of lysate from 10⁸ CFU/ml probiotic bacteria was added to the apical chamber of the Thincert™ we observed significant differences in the development of TEER between untreated and treated NHEK. These differences were strain dependent. In good agreement with the observation that lysates of *L. fermentum* reduce cell viability, we observed a significant decrease in TEER in NHEK treated with this lysate compared to untreated cells (Figure 17b, p<0.005). By contrast, lysates of *L. reuteri* initially increased the TEER at 24 hours post calcium switch, but this subsequently declined back to levels not significantly different to that of control, untreated cells (Figure 17c). *L. plantarum* also produced an increase in TEER over that of control cells. This increase was sustained for 48 hours post calcium switch. However, at 72 hours the TEER was not significantly different to that of untreated NHEK (Figure 17d). The largest increases in TEER were observed in NHEK treated with *B. longum reuterand L. rhamnosus* GG lysates. Lysates from both these strains produced increases in TEER that were ∼300ohms.cm² higher than in control cells (Figures 17e and f, p<0.05). Furthermore, these increases were sustained and the TEER in treated cells was still significantly greater in treated vs untreated NHEK at 72 hours post calcium switch. For these reasons, lysates of *B*. *longum reuter* and *L. rhamnosus* GG were taken forward for further investigation.

### B. longum reuter and L. rhamnosus GG produce dose dependent effects on TEER in keratinocytes.

To further define the effects of B. *longum reuter* and *L. rhamnosus* GG on TJ function in keratinocytes, we measured TEER in the presence of 100 µl lysate made from bacteria at 10⁶, 10⁴ and 10² CFU/ml. At 10⁶ CFU/ml, lysates from both strains were still able to produces TEERs that were significantly higher than in untreated cells (Figure 18a and b, p<0.005). Additionally, lysates of *L. rhamnosus* GG produced from 10⁴ CFU/ml were also able to elicit and increase in TEER (Figure 18b). However, lower concentrations of either strain resulted in lysates that were unable to produce TEERs significantly different to those of control cells (Figure 18a and b).

### Probiotic lysates modulate the expression of specific TJ proteins.

Evidence suggests that TJ function is often reflected in the expression levels of particular proteins involved in the complexes. The main TJ proteins expressed by keratinocytes include claudin 1, claudin 4, ZO-1 and occludin. Therefore, we used immunoblotting to investigate the expression of these proteins in untreated NHEK vs NHEK treated with probiotic lysates for 72 hours.

Treatment of NHEK with a lysate of B. *longum reuter,* produced significant increases in all four TJ proteins (Figure 19a and b). However, *L. rhamnosus* GG induced increases in the protein levels of claudin 1, occludin and ZO-1 only (Figure 19a and b). There was no significant change in the levels of claudin 4 in *L. rhamnosus* GG treated NHEK vs control cells. However, in general, increases in protein expression elicited by *L. rhamnosus* GG were greater than those induced by *B. longum reuter* (Figure 19b).

### Bifidobacterium longum reuter- induced modulation of TJ function is mediated via TLR2.

Keratinocytes sense the presence of bacteria via pattern recognition receptors such as toll like receptors (TLRs). Several lines of evidence in the gut have pointed to a relationship between TLR activation and changes in TJ barrier function. Additionally, recent work by Yuki et al, (53) demonstrated TLR-mediated augmentation of TJ function in keratinocytes in response to bacterial ligands such as peptidoglycan. Therefore, we naturally wondered whether the probiotic induced increases in TJ function were mediated by TLRs. Of particular interest was TLR2 because this is the major receptor for gram positive organisms (54).

To investigate this we incubated NHEK in the presence of a TLR2 neutralising antibody for 1 hour prior to the addition of probiotic lysates. The data in Figure 20a demonstrate that neutralisation of TLR2 abolished the rise in TEER elicited by both peptidoglycan (a well characterised TLR2 agonist) and B. *longum reuter.* However, a lysate of *L*. *rhamnosus* GG was still able to induce increases in TJ function (Figure 20a). Similarly, the increase in TJ protein expression induced by *B. longum reuter,* but not *L. rhamnosus* GG was prevented by neutralisation of TLR2 (Figure 20b).

### Discussion

Five different strains of lactobacilli were tested for their effect on TJ function. Of these, lysates of all but one of the strains, *L. fermentum,* were able to increase the TEER of keratinocytes. *L. fermentum* actually reduced the TEER which is probably related to our observation that this strain also reduced the viability of keratinocytes. The other 4 strains of lactobacilli all enhanced TJ function but with different efficacies. In this regard, *L. rhamnosus* GG and *B*. *longum reuter* produced greater, and more sustained increases in TEER than *L. plantarum* or *L. reuteri.*

These strain dependent effects are probably due to the expression of different proteins and carbohydrates by individual strains of lactobacilli. Indeed, the increase in TEER induced by *L. rhamonsus* GG and *B*. *longum reuter* exhibited dose dependent effects suggesting that a particular molecule(s) and receptive mechanism(s) is involved.

Both *L. rhamnosus* GG and *B*. *longum reuter* increased the expression of TJ proteins in keratinocytes. However, the particular subset of molecules was different in each case. The modulation of protein expression is almost certainly the mechanism by which these lysates increase the TJ barrier function of keratinocytes. Changes in the expression levels of claudins in particular have been shown many times previously to be linked to changes in barrier function.

The mechanism by which *B. longum reuter,* but not *L. rhamnosus GG* increases TJ protein expression and TEER is almost certainly associated with signalling through TLR2. This is demonstrated by two lines of evidence: 1) neutralisation of TLR2 abolishes B. *longum* reuter-induced increases in TEER and 2) the increase in TJ protein expression is also abolished by blocking TLR2. This is perhaps not surprising given that TLR2 is the major pattern recognition receptor for gram positive bacteria.

The link between the innate immune system and barrier regulation has only recently been established in the gut. Here, activation of TLRs can either increase or decrease epithelial tight junction function depending on the particular TLR activated.

The *L. rhamnosus* GG lysate appeared to elicit its effects via a mechanism independent of TLR2 because neutralisation of this receptor did not abolish the *L. rhamnosus* GG-induced increase in TJ function or protein expression. TLR2 is the major receptor for gram positive species and is activated by several different ligands from these bacteria such as lipteichoic acid. The observation that *L. rhamnosus* GG induced increases in TJ function does not appear to be mediated by TLR 2 may suggest that this lactobacillus possesses molecules that signal via alternative receptors.

Our data suggest that specific strains of Lactobacilli enhance TJ function in human primary keratinocytes. The barrier function of the epidermis is critical for life as terrestrial organisms and recent data has highlighted the essential role of TJs in the epidermal permeability barrier to water. Our data suggest that the augmentation of TJ barrier induced by probiotic bacterial lysates, could have a role in enhancing overall skin barrier function.

### REFERENCES

**1.** Aly, R., H. R. Shinefield, C. Litz, and H. I. Maibach. 1980. Role of Teichoic Acid in the Binding of Staphylococcus aureus to Nasal Epithelial Cells. The Journal of Infectious Diseases 141:463-465**.**
**2.** Baba, H., A. Masuyama, and T. Takano. 2006. Short Communication: Effects of Lactobacillus helveticus-Fermented Milk on the Differentiation of Cultured Normal Human Epidermal Keratinocytes. Journal of Dairy Science 89:2072-2075.
**3.** Backhed, F., R. E. Ley, J. L. Sonnenburg, D. A. Peterson, and J. I. Gordon. 2005. Host-Bacterial Mutualism in the Human Intestine. Science 307:1915-1920.
**4.** Balma-Mena, A., I. Lara-Corrales, J. Zeller, S. Richardson, M. J. McGavin, M. Weinstein, and E. Pope. 2011. Colonization with community-acquired methicillin-resistant Staphylococcus aureus in children with atopic dermatitis: a cross-sectional study. International Journal of Dermatology 50:682-688.
**5.** Banerjee, P., G. Merkel, and A. Bhunia. 2009. Lactobacillus delbrueckii ssp. bulgaricus B-30892 can inhibit cytotoxic effects and adhesion of pathogenic Clostridium difficile to Caco-2 cells. Gut Pathogens 1:8.
**6.** Bek-Thomsen, M., H. B. Lomholt, and M. Kilian. 2008. Acne is Not Associated with Yet-Uncultured Bacteria. Journal of Clinical Microbiology 46:3355-3360***.***
**7.** Bowler, P. G., B. I. Duerden, and D. G. Armstrong. 2001. Wound Microbiology and Associated Approaches to Wound Management. Clin. Microbiol. Rev. 14:244-269.
**8.** Burkhart, C. N., and C. G. Burkhart. 2003. Microbiology's principle of biofilms as a major factor in the pathogenesis of acne vulgaris. International Journal of Dermatology 42:925-927.
**9.** Caglar, E., S. Kavaloglu Cildir, S. Ergeneli, N. Sandalli, and S. Twetman. 2006. Salivary mutans streptococci and lactobacilli levels after ingestion of the probiotic bacterium Lactobacillus reuteri ATCC 55730 by straws or tablets. Acta Odontologica Scandinavica 64:314-318.
**10.** Chen, X., J. Xu, J. Shuai, J. Chen, Z. Zhang, and W. Fang. 2007. The S-layer proteins of Lactobacillus crispatus strain ZJ001 is responsible for competitive exclusion against Escherichia coli O157:H7 and Salmonella typhimurium. International Journal of Food Microbiology 115:307-312.
**11.** Clarke, S. R., S. J. Foster, and K. P. Robert. 2006. Surface Adhesins of Staphylococcus aureus, p. 187-224. Advances in Microbial Physiology, vol. Volume 51. Academic Press.
**12.** Coconnier, M. H., M. F. Bernet, S. Kerneis, G. Chauviere, J. Fourniat, and A. L. Servin. 1993. Inhibition of adhesion of enteroinvasive pathogens to human intestinal Caco-2 cells by Lactobacillus acidophilus strain LB decreases bacterial invasion. FEMS microbiology letters 110:299-306.
**13.** Cotter, P. D., C. Hill, and R. P. Ross. 2005. Bacteriocins: developing innate immunity for food. Nat Rev Micro 3:777-788.
**14.** Deepika, G., D. Charalampopoulos, I. L. Allen, S. Sima, and M. G. Geoffrey. 2010. Surface and Adhesion Properties of Lactobacilli, p. 127-152. Advances in Applied Microbiology, vol. Volume 70. Academic Press.
**15.** Di Marzio, L., B. Cinque, C. De Simone, and M. G. Cifone. 1999. Effect of the Lactic Acid Bacterium Streptococcus thermophilus on Ceramide Levels in Human Keratinocytes In Vitro and Stratum Corneum In Vivo. 113:98-106.
**16.** Edmond, M. B., S. E. Wallace, D. K. McClish, M. A. Pfaller, R. N. Jones, and R. P. Wenzel. 1999. Nosocomial Bloodstream Infections in United States Hospitals: A Three-Year Analysis. Clinical Infectious Diseases 29:239-244.
**17.** FAO/WHO. 2002. Guidelines for the Evaluation of Probiotics in Food: Report of a Joint FAO/WHO Working Group on Drafting Guidelines for the Evaluation of Probiotics in Food.
**18.** Gan, B. S., J. Kim, G. Reid, P. Cadieux, and J. C. Howard. 2002. Lactobacillus fermentum RC-14 Inhibits Staphylococcus aureus Infection of Surgical Implants in Rats. The Journal of Infectious Diseases 185:1369-1372.
**19.** Granato, D., F. Perotti, I. Masserey, M. Rouvet, M. Golliard, A. Servin, and D. Brassart. 1999. Cell Surface-Associated Lipoteichoic Acid Acts as an Adhesion Factor for Attachment of Lactobacillus johnsonii La1 to Human Enterocyte-Like Caco-2 Cells. Appl. Environ. Microbiol. 65:1071-1077.
**20.** Guéniche, A., P. Bastien, J. M. Ovigne, M. Kermici, G. Courchay, V. Chevalier, L. Breton, and I. Castiel-Higounenc. 2010. Bifidobacterium longum lysate, a new ingredient for reactive skin. Experimental Dermatology 19:e1-e8.
**21.** Heinemann, C., J. E. T. van Hylckama Vlieg, D. B. Janssen, H. J. Busscher, H. C. van der Mei, and G. Reid. 2000. Purification and characterization of a surface-binding protein from Lactobacillus fermentum RC-14 that inhibits adhesion of Enterococcus faecalis 1131. FEMS Microbiology Letters 190:177-180.
**22.** Helgeland, L., J. T. Vaage, B. Rolstad, T. Midtvedt, and P. Brandtzaeg. 1996. Microbial colonization influences composition and T-cell receptor V beta repertoire of intraepithelial lymphocytes in rat intestine. Immunology 89:494-501.
**23.** Holder, I. A., and S. T. Boyce. 1994. Agar well diffusion assay testing of bacterial susceptibility to various antimicrobials in concentrations non-toxic for human cells in culture. Burns 20:426-429.
**24.** Jones, M., J. Ganopolsky, A. Labbe, and S. Prakash. 2010. A novel nitric oxide producing probiotic patch and its antimicrobial efficacy: preparation and in vitro analysis. Applied Microbiology and Biotechnology 87:509-516.
**25.** Karska-Wysocki, B., M. Bazo, and W. Smoragiewicz. 2010. Antibacterial activity of Lactobacillus acidophilus and Lactobacillus casei against methicillin-resistant Staphylococcus aureus (MRSA). Microbiological Research 165:674-86**.**
**26.** Kintarak, S., S. P. Nair, P. M. Speight, and S. A. Whawell. 2004. A recombinant fragment of the fibronectin-binding protein of Staphylococcus aureus inhibits keratinocyte migration. Archives of Dermatological Research 296:250-257.
**27.** Kintarak, S., S. A. Whawell, P. M. Speight, S. Packer, and S. P. Nair. 2004. Internalization of Staphylococcus aureus by Human Keratinocytes. Infect. Immun. 72:5668-5675.
**28.** Kluytmans, J., A. Van Belkum, and H. Verbrugh. 1997. Nasal carriage of Staphylococcus aureus: Epidemiology, underlying mechanisms, and associated risks. Clinical Microbiology Reviews 10:505-520.
**29.** Krut, O., O. Utermohlen, X. Schlossherr, and M. Kronke. 2003. Strain-Specific Association of Cytotoxic Activity and Virulence of Clinical Staphylococcus aureus Isolates. Infect. Immun. 71:2716-2723.
**30.** Lai, Y., A. L. Cogen, K. A. Radek, H. J. Park, D. T. MacLeod, A. Leichtle, A. F. Ryan, A. Di Nardo, and R. L. Gallo. 2010. Activation of TLR2 by a Small Molecule Produced by Staphylococcus epidermidis Increases Antimicrobial Defense against Bacterial Skin Infections. J Invest Dermatol 130:2211-2221.
**31.** Lai, Y., A. Di Nardo, T. Nakatsuji, A. Leichtle, Y. Yang, A. L. Cogen, Z.-R. Wu, L. V. Hooper, R. R. Schmidt, S. von Aulock, K. A. Radek, C.-M. Huang, A. F. Ryan, and R. L. Gallo. 2009. Commensal bacteria regulate Toll-like receptor 3-dependent inflammation after skin injury. Nat Med 15:1377-1382.
**32.** Lee, Y.-K., K.-Y. Puong, A. C. Ouwehand, and S. Salminen. 2003. Displacement of bacterial pathogens from mucus and Caco-2 cell surface by lactobacilli. Journal of Medical Microbiology 52:925-930.
**33.** Mack, D. R., S. Michail, S. Wei, L. McDougall, and M. A. Hollingsworth. 1999. Probiotics inhibit enteropathogenic E. coli adherence in vitro by inducing intestinal mucin gene expression. Am J Physiol Gastrointest Liver Physiol 276:G941-950.
**34.** Maudsdotter, L., H. Jonsson, S. Roos, and A.-B. Jonsson. 2011. Lactobacilli Reduce Cell Cytotoxicity Caused by Streptococcus pyogenes by Producing Lactic Acid that Degrades the Toxic Component Lipoteichoic Acid. Antimicrobial agents and chemotherapy 55:1622-88.
**35.** Mempel, M., T. Schmidt, S. Weidinger, C. Schnopp, T. Foster, J. Ring, and D. Abeck. 1998. Role of Staphylococcus aureus Surface-Associated Proteins in the Attachment to Cultured HaCaT Keratinocytes in a New Adhesion Assay. Journal of Investigative Dermatology 111:452-456.
**36.** Mempel, M., C. Schnopp, M. Hojka, H. Fesq, S. Weidinger, M. Schaller, H. C. Korting, J. Ring, and D. Abeck. 2002. Invasion of human keratinocytes by Staphylococcus aureus and intracellular bacterial persistence represent haemolysin-independent virulence mechanisms that are followed by features of necrotic and apoptotic keratinocyte cell death. British Journal of Dermatology 146:943-951.
**37.** Miyoshi, Y., S. Okada, T. Uchimura, and E. Satoh. 2006. A Mucus Adhesion Promoting Protein, MapA, Mediates the Adhesion of Lactobacillus reuteri to Caco-2 Human Intestinal Epithelial Cells. Bioscience, Biotechnology, and Biochemistry 70:1622-1628.
**38.** Nikawa, H., S. Makihira, H. Fukushima, H. Nishimura, Y. Ozaki, K. Ishida, S. Darmawan, T. Hamada, K. Hara, A. Matsumoto, T. Takemoto, and R. Aimi. 2004. Lactobacillus reuteri in bovine milk fermented decreases the oral carriage of mutans streptococci. International Journal of Food Microbiology 95:219-223.
**39.** Ouwehand, A. C., E. Isolauri, P. V. Kirjavainen, S. T. ölkkö, and S. J. Salminen. 2000. The mucus binding of Bifidobacterium lactis Bb12 is enhanced in the presence of Lactobacillus GG and L. delbrueckii subsp. bulgaricus. Letters in Applied Microbiology 30:10-13.
**40.** Peral, M. C., M. A. Huaman Martinez, and J. C. Valdez. 2009. Bacteriotherapy with Lactobacillus plantarum in burns. International Wound Journal 6:73-81.
**41.** Peral, M. C., M. M. Rachid, N. M. Gobbato, M. A. H. Martinez, and J. C. Valdez. 2009. Interleukin-8 production by polymorphonuclear leukocytes from patients with chronic infected leg ulcers treated with Lactobacillus plantarum. Clinical Microbiology and Infection 16:281-286.
**42.** Rembacken, B. J., A. M. Snelling, P. M. Hawkey, D. M. Chalmers, and A. T. R. Axon. 1999. Non-pathogenic Escherichia coli versus mesalazine for the treatment of ulcerative colitis: a randomised trial. The Lancet 354:635-639.
**43.** Ron, E. Z., and E. Rosenberg. 2001. Natural roles of biosurfactants. Environmental Microbiology 3:229-236.
**44.** Shornikova, A. V., I. A. Casas, E. Isolauri, H. Mykkanen, and T. Vesikari. 1997. Lactobacillus reuteri as a therapeutic agent in acute diarrhoea in young children. J Pediatr Gastroenterol Nutr 24:399 - 404.
**45.** Strober, W. 1997. Trypan blue exclusion test of cell viability, p. Appendix 3B. Current Protocols in Immunology. John Wiley & Sons, Inc.
**46.** Valdéz, J. C., M. C. Peral, M. Rachid, M. Santana, and G. Perdigón. 2005. Interference of Lactobacillus plantarum with Pseudomonas aeruginosa in vitro and in infected burns: the potential use of probiotics in wound treatment. Clinical Microbiology & Infection 11:472-479.
**47.** Vanderhoof, J. A., D. B. Whitney, D. L. Antonson, T. L. Hanner, J. V. Lupo, and R. J. Young. 1999. Lactobacillus GG in the prevention of antibiotic-associated diarrhoea in children. The Journal of Pediatrics 135:564-568.
**48.** Wanke, I., H. Steffen, C. Christ, B. Krismer, F. Gotz, A. Peschel, M. Schaller, and B. Schittek. 2011. Skin Commensals Amplify the Innate Immune Response to Pathogens by Activation of Distinct Signalling Pathways. J Invest Dermatol 131:382-390.
**49.** Zarate, G., and M. E. Nader-Macias. 2006. Influence of probiotic vaginal lactobacilli on in vitro adhesion of urogenital pathogens to vaginal epithelial cells. Lett Appl Microbiol 43:174 - 180.
**50.** Prince T, Mcbain AJ and O'Neill CA. 2012. Lactobacillus reuteri protects epidermal keratinocytes from Syaphylococcus auresu induced cell death by competitive exclusion. Appl. Environ. Microbiol. 78(15):5119-26.
**51.** McLaughlin J. Padfield PJ, Burt JP and O'Neill CA. 2004. Ochratoxin A increases permeability through tight junctions by removal of specific claudin isoforms. Am J. Physiol. Cell Physiol.287(5):C1412-7.
**52.** Laemmli UK 1970. Cleavage of structural proteins during the assembly of the head of bacteriophage T4. Nature; 227: 680-685.
**53.** Yuki T, Yoshida H, Akazawa Y, Komiya A, Sugiyama Y, Inoue S. 2011. Activation of TLR2 enhances tight junction barrier in epidermal keratinocytes. J Immunol 187(6):3230-7.
**54.** Fournier B and Philpott DJ. 2005. Recognition of Staphylococcus aureus by the innate immune system.Clin Microbiol Rev. 18(3):521-40
**55.** Tsukita S and Furuse M. 2002 Claudin based barrier in simple and stratified cellular sheets. Curr Opin. Cell Biol. 14(5):531-6
**56.** Miyauchi E, O'Callaghan J, Butto LF, Hurley G, Melgar S, Tanabe S, Shanahan F, Nally K, O'Toole PW. 2012. Mechanism of protection of transepithelial barrier function by Lactobacillus salivarius: strain dependence and attenuation by bacteriocin production. Am J Physiol Gastrointest Liver Physiol. 303(9):G1029-41.
**57.** Seth A, Yan F, Polk DB and Rao RK. 2008. Probiotics ameliorate the hydrogen peroxide-induced epithelial barrier disruption by a PKC- and MAP kinase-dependent mechanism. Am J Physiol Gastrointest Liver Physiol. 294(4):G1060-9
**58.** Krause G, Winkler L, Mueller SL, Haseloff RF, Piontek J, Blasig IE.2008. Structure and function of claudins. Biochim Biophys Acta 1778(3):631-45.
**59.** Coyne, C.B., Gambling, T.M., Boucher, R.C., Carson, J.L. and Johnson, L.G. 2003. Role of claudin interactions in airway junctional permeability. Am J Physiol Lung Cell Physiol 285(5):L1166-78
**60.** Michikawa H, Fujita-Yoshigaki J, Sugiya H. 2008. Enhancement of barrier function by overexpression of claudin-4 in tight junctions of submandibular gland cells.Cell Tiss Res 334(2):255-64.
61. Ko JA, Murata S and Nishida T 2009. Up-regulation of the tight-junction protein ZO-1 by substance P and IGF-1 in A431 cells. Cell Biochem Funct. 27(6):388-94.
62. Moran GW, O'Neill CA and McLaughlin JT 2012. GLP-2 enhances barrier formation and attenuates TNFa-induced changes in a Caco-2 cell model of the intestinal barrier.Regul Pept. 178(1-3):95-101.
63. Furuse M, Hata M, Furuse K, Yoshida Y, Haratake A, Sugitani Y, Noda T, Kubo A, Tsukita S. 2002. Claudin-based tight junctions are crucial for the mammalian epidermal barrier: a lesson from claudin-1-deficient mice. J Cell Biol. 156(6):1099-111.

## Claims

1. Use of a filtered probiotic bacterium lysate for cosmetically, non-therapeutically increasing skin barrier function in a subject, wherein the probiotic bacterium lysate is from a strain of *Lactobacillus rhamnosus, Lactobacillus reuteri,* or *Bifidobacterium longum* and wherein the use comprises administering the lysate topically to the skin of the subject.

2. The use according to claim 1 wherein the increased skin barrier function comprises an increased viability of keratinocytes and/or the tight junction barrier.

3. The use according to claim 1 or 2 wherein the probiotic bacterium lysate is from a strain of *Lactobacillus rhamnosus.*

4. The use according to claim 3 wherein the *Lactobacillus rhamnosus* strain comprises *Lactobacillus rhamnosus* GG or *Lactobacillus rhamnosus* ATCC 53103.

5. The use according to claim 1 or 2 wherein the probiotic bacterium lysate is from a strain of *Lactobacillus reuteri.*

6. The use according to claim 5 wherein the *Lactobacillus reuteri* strain comprises *Lactobacillus reuteri* ATCC 55730.

7. The use according to claim 1 or 2 wherein the probiotic bacterium lysate is from a strain of *Bifidobacterium longum.*

8. The use according to claim 7 wherein the *Bifidobacterium longum* strain comprises *Bifidobacterium longum* ATCC 51870.

9. The use according to any preceding claim wherein the lysate is filter sterilised.

10. The use according to any preceding claim wherein the lysate comprises a cell free supernatant of the probiotic bacterium.
